# FASCICULE DE BREVET EUROPEEN

(11) **EP 3 233 779 B1**
(45) Date de publication et mention de la délivrance du brevet: **19.07.2023**
(21) Numéro de dépôt: 15828347.3
(22) Date de dépôt: 16.12.2015
(51) Int. Cl.: C07C 45/72, C07C 45/75, C07C 49/12, C07C 49/20, C07C 49/21

(54) **NOUVEAU PROCEDE DE PREPARATION D'INTERMEDIAIRES DE SYNTHESE UTILISANT DES PRODUITS D'ORIGINE NATURELLE ET APPLICATION DES INTERMEDAIRES OBTENUS**
NEUARTIGES VERFAHREN ZUR HERSTELLUNG VON SYNTHESEZWISCHENPRODUKTEN UNTER VERWENDUNG VON PRODUKTEN NATÜRLICHEN URSPRUNGS UND VERWENDUNG DER GEWONNENEN ZWISCHENPRODUKTE
NOVEL PROCESS FOR PREPARING SYNTHESIS INTERMEDIATES USING PRODUCTS OF NATURAL ORIGIN AND USE OF THE INTERMEDIATES OBTAINED

(30) Priorité: 16.12.2014 FR 1462548
(43) Date de publication de la demande: 25.10.2017
(73) Titulaire: Centre National de la Recherche Scientifique (C.N.R.S.), 75794 Paris Cedex 16 (FR); Université de Montpellier, 34090 Montpellier (FR)
(72) Inventeur: GRISON, Claude, 34170 Castelnau-le-Lez (FR); ESCANDE, Vincent, 34000 Montpellier (FR); STANOVYCH, Andrii, 34830 Jacou (FR)
(74) Mandataire: Lavoix
(86) Numéro de dépôt international: PCT/FR2015/053559
(87) Numéro de publication internationale: WO 2016/097612

(56) Documents cités:
- GB-A- 1 465 668
- AKIRA MIYASHITA ET AL: "Catalytic Action of Azolium Salts. VI. Preparation of Benzoins and Acyloins by Condensation of Aldehydes Catalyzed by Azolium Salts.", CHEMICAL AND PHARMACEUTICAL BULLETIN, vol. 42, no. 12, 1 janvier 1994 (1994-01-01), pages 2633-2635, XP55728066, JP ISSN: 0009-2363, DOI: 10.1248/cpb.42.2633
- STETTER: "ACYLOIN CONDENSATION BY THIAZOLIUM ION CATALYSIS: BUTYROIN", ORGANIC SYNTHESES, vol. 62, 1 janvier 1984 (1984-01-01), page 170, XP055729059, US ISSN: 0078-6209, DOI: 10.15227/orgsyn.062.0170
- R. S. PIMPIM ET AL: "An Efficient One-Step Thiamine Catalyzed Synthesis of Furil and Its Analogues", SYNTHETIC COMMUNICATIONS, vol. 27, no. 5, 1 mars 1997 (1997-03-01), pages 811-815, XP055728898, PHILADELPHIA, PA; US ISSN: 0039-7911, DOI: 10.1080/00397919708004200
- HERMANN STETTER: "Catalyzed addition of aldehydes to activated double bonds - A new synthetic approach", ANGEWANDTE CHEMIE INTERNATIONAL EDITION, WILEY - V C H VERLAG GMBH & CO. KGAA, DE, vol. 15, no. 11, 1 novembre 1976 (1976-11-01), pages 639-712, XP002747528, ISSN: 1433-7851, DOI: 10.1002/ANIE.197606391
- NIALL W.A. GERAGHTY: "Synthesis of 2-alkyl-2-cyclopenten-1-ones. A versatile kinetic alkylation-ozonolysis procedure for the preparation of gamma-ketoaldehydes", SYNTHESIS, vol. 8, 1 janvier 1989 (1989-01-01), pages 603-607, XP055222287,
- HERMANN STETTER ET AL: "Intramolekulare Aldol-Reaktion von [delta],[epsilon]-Ungesättigten [gamma]-Diketonen", SYNTHESIS, vol. 1979, no. 03, 1 janvier 1979 (1979-01-01), pages 187-188, XP055222261, STUTTGART, DE. ISSN: 0039-7881, DOI: 10.1055/s-1979-28609
- AKIRA MIYASHITA ET AL: "Catalytic Action of Azolium Salts. VI. Preparation of Benzoins and Acyloins by Condensation of Aldehydes Catalyzed by Azolium Salts.", CHEMICAL AND PHARMACEUTICAL BULLETIN, vol. 42, no. 12, 1 January 1994 (1994-01-01), pages 2633-2635, XP55728066, JP ISSN: 0009-2363, DOI: 10.1248/cpb.42.2633
- STETTER: "ACYLOIN CONDENSATION BY THIAZOLIUM ION CATALYSIS: BUTYROIN", ORGANIC SYNTHESES, vol. 62, 1 January 1984 (1984-01-01), page 170, XP055729059, US ISSN: 0078-6209, DOI: 10.15227/orgsyn.062.0170
- R. S. PIMPIM ET AL: "An Efficient One-Step Thiamine Catalyzed Synthesis of Furil and Its Analogues", SYNTHETIC COMMUNICATIONS, vol. 27, no. 5, 1 March 1997 (1997-03-01), pages 811-815, XP055728898, PHILADELPHIA, PA; US ISSN: 0039-7911, DOI: 10.1080/00397919708004200

## Description

L'industrie de la chimie de synthèse est en train de vivre un changement de paradigme. L'engouement actuel du "tout naturel", "tout bio" créée un nouveau marché orienté vers les produits "verts". Parallèlement, le durcissement de la réglementation REACH et la mauvaise perception de la chimie par l'opinion publique convergent vers une nouvelle approche de la chimie, qui se veut aujourd'hui éco-responsable. Cette évolution rapide se concrétise par le développement de nouveaux procédés de synthèse respectueux de l'environnement et axés sur la préparation de produits bio-sourcés. Cette situation est un véritable défi scientifique et technique, qui nécessite innovation et intégration de nouvelles stratégies de synthèse. Les industries cosmétiques sont les premières à s'inscrire dans cette démarche et manifestent clairement leur intérêt pour les procédés de chimie organique écologique et bio-inspirés. Nous décrivons ici une réaction de Stetter totalement revisitée, qui s'appuie sur l'utilisation de réactifs et catalyseurs totalement biosourcés. Cette nouvelle approche verte de la réaction de Stetter permet un accès beaucoup plus efficace à des dérivés utilisables en synthèse organique et notamment de dérivés dicarbonylés 1,4 (dicétones et céto aldéhydes) et aux des cétones cycliques correspondantes, telles que les dérivés jasmoniques. L'originalité de la méthodogie repose sur l'utilisation conjointe de deux vitamines, la vitamine C et la vitamine B1. Aucune base toxique, corrosive, synthétique n'est utile.

La méthodologie mise au point par Hermann Stetter en 1976 a été décrite par exemple dans les publications suivantes : H. Stetter, Angewandte Chemie International Edition in English 1976, 15, 639-647; H. Stetter, H.-J. Bender, Angewandte Chemie International Edition in English 1978, 17, 131-131; H. Stetter, H. Kuhlmann, Tetrahedron Letters 1974, 15, 4505-4508; H. Stetter, H. Kuhlmann, W. Haese, Organic Synthèses 1987, 25, 26 ; H. Stetter, H. Kuhlmann, Organic Reactions 1991, 40, 400. A. Miyashit et al. Chem.Pharm.Bull. 1994, 42 (12), 2633-2635 divulgue la condensation acyloïne d'heptanale Cette réaction permet notamment la synthèse de dicétones 1,4 selon une réaction « umpolung » (inversion de polarité). Cette réaction est directement inspirée par le rôle biologique de la thiamine (vitamine B1), capable de transformer temporairement un aldéhyde (électrophile) en nucléophile. Cette réaction est habituellement conduite en utilisant un thiazolium synthétique dérivé de la thiamine (le 3-benzyl-5-(2-hydroxyethyl)-4-methyl-1,3-thiazolium chloride), mais comportant moins de groupes fonctionnels que la thiamine, groupes fonctionnels qui pourraient s'avérer gênants pour le bon déroulement de la réaction.

Par ailleurs, ce thiazolium doit être déprotoné pour attaquer l'aldéhyde, ce qui explique l'utilisation d'une base, classiquement la triéthylamine. Cette réaction est conduite dans différents solvants, principalement l'éthanol ou le diméthylformamide.

La présente invention a consisté à mettre au point une version totalement biosourcée de cette réaction, ce qui nous a donc conduit à choisir l'éthanol comme solvant et à remplacer le thiazolium de Stetter par la véritable thiamine, produit d'origine naturel, bien que celle-ci soit plus difficile à faire réagir.

Le but de la présente invention étant d'utiliser dans les synthèses proposées, des produits dont une base d'origine naturelle, un grand nombre de bases potentielles ont été testées, notamment les bases suivantes :
_ hydroxydes obtenus par hydratation des cendres ou par action de la soude sur les cendres de plantes accumulatrices de Zn décrites dans la demande WO 2013/150197 (réaction testée sous chauffage conventionnel et sous activation micro-ondes),
_ hydroxydes obtenus par hydratation des cendres ou par action de la soude sur les cendres des plantes accumulatrices de Zn décrites dans la demande WO 2011/064487 (réaction testée sous chauffage conventionnel et sous activation micro-ondes),
_ hydroxydes obtenus par hydratation des cendres ou par action de la soude sur les cendres des plantes accumulatrices de Ni décrites dans la demande WO 2011/064487 (réaction testée sous chauffage conventionnel et sous activation micro-ondes),
_ citrates obtenus par action de l'acide citrique sur les cendres des plantes accumulatrices de Zn décrites dans la demande WO 2011/064487,
_ acétates obtenus par action de l'acide acétique sur les cendres des plantes accumulatrices de Zn décrites dans la demande WO 2011/064487,
_ alumine basique,
_ fluorure de Na supporté sur alumine,
_ fluorures obtenus par action de fluorures alcalins (Na, K) sur les chlorures métalliques obtenus par action de l'acide chlorhydrique sur les cendres des plantes accumulatrices de Zn décrites dans la demande WO 2011/064487, supportés sur alumine.
_ urée
_ CaCO₃
_ NaHCO₃
< arginine
_ complexe cuivre-arginine
_ Ca(OH)₂
_ cystéine
_ asparagine
_ histidine
_ glycine
_ alanine
_ glutamate de sodium
_ glutamine
_ arginine
_ thréonine
_ lysine
_ sérine
_ allantoïne
_ riboflavine
_ chitosan
_ leucine
_ gluconate de potassium
_ acétate de sodium hydraté
_ citrate de sodium hydraté
_ salicylate de sodium
_ oxalate de sodium
_ oxalate de calcium
_ citrate de calcium
_ pyrrolidine.

Les essais qui ont été réalisés avec ces molécules basiques d'origine naturelle ont conduit les inventeurs à en exclure l'utilisation. C'est notamment le cas de l'utilisation d'hydroxydes, de carbonates, d'hydrogénocarbonates et d'acides aminés (tels que l'histidine ou l'arginine, choisies pour leurs pKa), en raison de l'absence totale de réaction.

Certains carboxylates ont montré une légère réactivité, ce qui avait été effectivement souligné par Stetter pour l'acétate de sodium.

Cependant, le rendement restait modeste avec l'acétate de sodium (55 %) dans le cas où le thiazolium utilisé (thiamine naturelle) ne possède pas la même réactivité que la molécule utilisée par Stetter (chlorure de 3-benzyl-5-(2-hydroxyethyl)-4-methyl-1,3-thiazolium).

D'autres carboxylates naturels se sont avérés relativement efficaces (tartrate de sodium-potassium, succinate de sodium) mais sans que le rendement global ne dépasse 30 %.

Les inventeurs se sont alors tournés vers une autre molécule naturelle, l'ascorbate de sodium.

Cette molécule, la vitamine C est habituellement utilisée comme réducteur en synthèse organique.

Son utilisation en tant que réactif basique n'a jamais été décrite.

Son utilisation en remplacement de la triéthylamine dans la réaction de Stetter, combinée à la vitamine B1 (thiamine), atteint 92 % dans le cas de la réaction mettant en jeu les substrats utilisés initialement par Stetter (heptanal et 3-buten-2-one), ce qui dépasse les 75 % décrits par cet auteur.

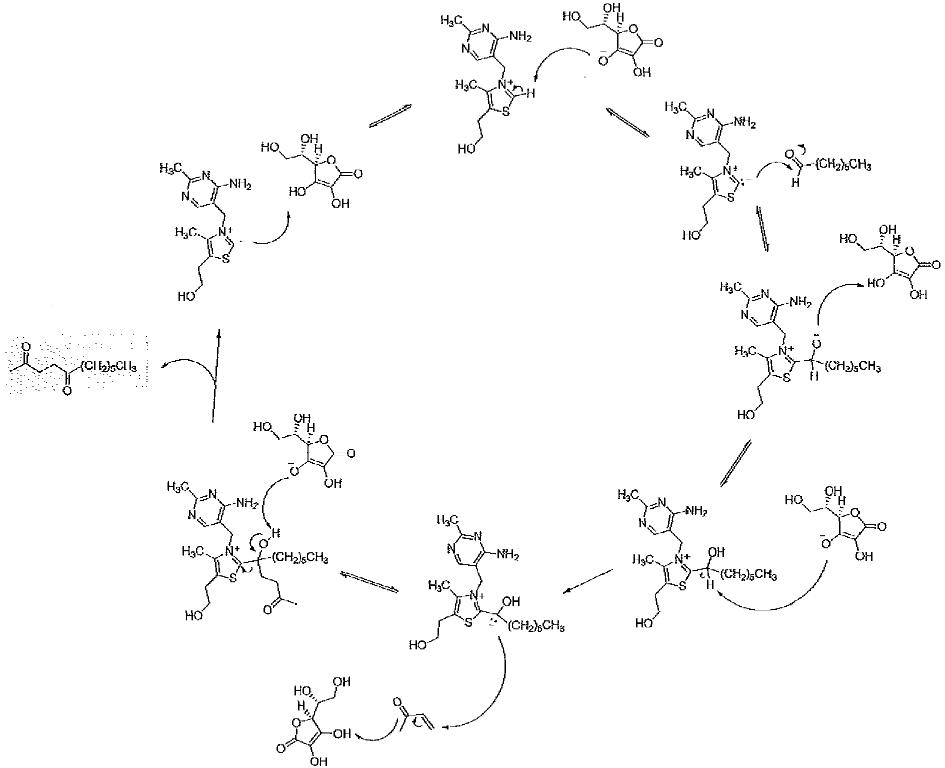

Cette méthodologie différente des conditions originales constitue donc une alternative totalement verte et d'origine naturelle, permettant l'accès à des dérivés carbonylés possédant un groupe attracteur en position 4, tels que des dicétones 1,4 avec d'excellentes performances.

La présente invention a donc pour objet un procédé de préparation d'un produit de formule I : 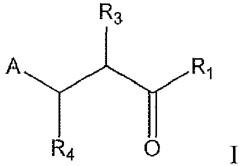 dans laquelle A représente un radical choisi parmi :
- CO-R₂
- CO-O-R₂a
- CN
- C(O)-N Ra R'a
- CH- (CO₂Rb)₂

R₁ représente un atome d'hydrogène ou un radical alkyle ou alkylène linéaire ou ramifié ayant au plus 12 atomes de carbone soit R₁ représente un radical cycloalkyle saturé ou insaturé ayant de 3 à 7 atomes de carbone et comportant éventuellement un ou plusieurs hétéroatomes choisis parmi les atomes d'azote, de soufre ou d'oxygène, soit R₁ représente un radical aryle carbocyclique ou hétérocyclique, chacun de ces radicaux alkyle, alkylène, aryle ou cycloalkyle étant éventuellement substitués,
R₂ représente un radical alkyle linéaire ou ramifié ayant de 1 à 12 atomes de carbone éventuellement substitué soit R₂ représente un radical cycloalkyle saturé ou insaturé ayant de 3 à 7 atomes de carbone et comportant éventuellement un ou plusieurs hétéroatomes choisis parmi les atomes d'azote, de soufre ou d'oxygène soit R₂ représente un radical aryle carbocyclique ou hétérocyclique chacun de ces radicaux alkyle, aryle ou cycloalkyle étant éventuellement substitués,
R₂a représente un radical alkyle linéaire ou ramifié ayant de 1 à 12 atomes de carbone
Ra et R'a identiques ou différents sont choisis parmi radicaux alkyle ou alkoxy linéaires ou ramifiés ayant de 1 à 12 atomes de carbone, étant entendu que Ra et R'a ne peuvent pas représenter simultanément chacun un radical alkoxy linéaire ou ramifié ayant de 1 à 12 atomes de carbone
Rb est choisi parmi les radicaux alkyles linéaires ou ramifiés ayant de 1 à 12 atomes de carbone
R₃ représente un atome d'hydrogène, alkyle ou alkylène linéaire ou ramifié ayant au plus 6 atomes de carbone soit R₃ représente un radical aryle carbocyclique ou hétérocyclique, chacun de ces radicaux alkyle, alkylène ou aryle étant éventuellement substitués,
ou les radicaux R₂ et R₃ sont liés entre eux pour former un cycle ayant de 5 à 7 atomes de carbone, ladite chaine comportant éventuellement un ou plusieurs hétéroatomes choisis parmi les atomes d'azote, de soufre ou d'oxygène,
R₄ est un atome d'hydrogène ou est choisi parmi les radicaux alkyle éventuellement substitués, acylamido ayant de 2 à 12 atomes de carbone, carboxylester, de préférence R₄ représente un atome d'hydrogène,
caractérisé en ce que l'on fait agir un produit de formule II : 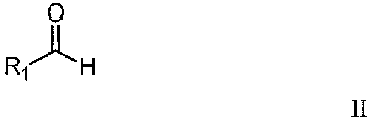
avec un produit de formule III : 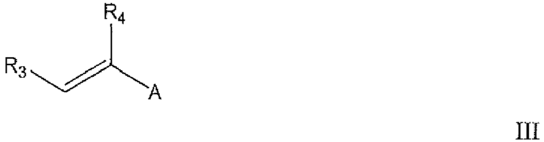
en présence d'une part d'un sel de thiazolium, d'un sel d'1,3-imidazolium ou d'un sel de 1,2,4-triazolium, notamment d'un sel de thiazolium ou d'un sel de 1,2,4-triazolium, de préférence d'un sel de thiazolium et d'autre part d'acide ascorbique sous forme libre ou salifiée ou d'un composé comprenant un motif 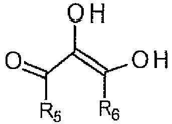
dans lequel R₅ et R₆ représentent indépendamment l'un de l'autre un atome d'hydrogène, un radical alkyle linéaire ou ramifié ayant de 1 à 12 atomes de carbone ou R₅ et R₆ sont liés entre eux pour former un cycle ayant de 3 à 7 chaînons, ledit alkyle ou ledit cycle étant éventuellement substitué par un ou plusieurs hétéroatomes, notamment choisi parmi O, N et S, notamment une réductone, de préférence le 2-hydroxypropanedial ou la 2,3-dihydroxycyclopent-2-en- 1-one ou l'acide croconique dans un solvant organique.

De manière avantageuse, la réaction est mise en oeuvre avec une cétone α,β-insaturée, c'est-à-dire un avec un produit de formule III : 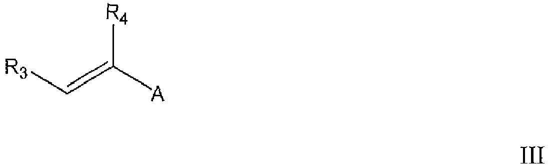 dans laquelle A représente un radical CO-R₂.

De préférence, la réaction est mise en oeuvre en présence d'une part d'un sel de thiazolium, ou d'un sel de 1,2,4-triazolium, de préférence d'un sel de thiazolium et d'autre part d'acide ascorbique sous forme libre ou salifiée ou d'un composé comprenant un motif 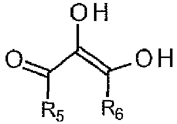 dans lequel R₅ et R₆ représentent indépendamment l'un de l'autre un atome d'hydrogène, un radical alkyle linéaire ou ramifié ayant de 1 à 12 atomes de carbone ou R₅ et R₆ sont liés entre eux pour former un cycle ayant de 3 à 7 chaînons, ledit alkyle ou ledit cycle étant éventuellement substitué par un ou plusieurs hétéroatomes, notamment choisi parmi O, N et S, notamment une réductone, de préférence le 2-hydroxypropanedial ou la 2,3-dihydroxycyclopent-2-en- 1-one ou l'acide croconique dans un solvant organique.

Le composé organique comportant au moins un cycle thiazolium est par exemple un composé de formule générale A suivante : 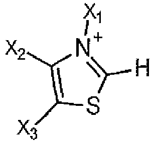 dans laquelle :
X₁ représente un groupement alkyle en C₁ à C₆, aryle en C₅ à C₁₀, notamment phényle, biphényle ou naphthyle, alkyle en C₁ à C₆ aryle en C₅ à C₁₀, notamment benzyle, hétéroaryle en C₅ à C₁₀, notamment pyridyle ou pyrimidyle, lesdits groupements alkyle, aryle, alkyl-aryle et hétéroaryle pouvant être substitués par un ou plusieurs hétéroatomes, notamment choisi parmi N, O ou S,
X₂ représente hydrogène, un groupement alkyle en C₁ à C₆, notamment méthyle, éthyle ou propyle, aryle en C₅ à C₁₀, notamment phényle, biphényle ou naphthyle, alkyle en C₁ à C₆ aryle en C₅ à C₁₀, notamment benzyle, hétéroaryle en C₅ à C₁₀, notamment pyridyle ou pyrimidyle, lesdits groupements alkyle, aryle, alkyl-aryle et hétéroaryle pouvant être substitués par un ou plusieurs hétéroatomes, notamment choisi parmi N, O ou S,
X₁ représente un groupement alkyle en C₁ à C₆, aryle en C₅ à C₁₀, notamment phényle, biphényle ou naphthyle, alkyle en C₁ à C₆ aryle en C₅ à C₁₀, notamment benzyle, hétéroaryle en C₅ à C₁₀, notamment pyridyle ou pyrimidyle, lesdits groupements alkyle, aryle, alkyl-aryle et hétéroaryle pouvant être substitués par un ou plusieurs hétéroatomes, notamment choisi parmi N, O ou S,
Le composé organique comportant au moins un cycle thiazolium est avantageusement de formule A1 suivante : 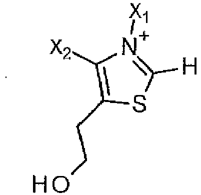
dans laquelle X₁ est tel que défini ci-dessus, avantageusement un hétéroaryle ou un alkyl-aryle,
X₂ est tel que défini ci-dessus, avantageusement un alkyle.

De préférence, le composé organique comportant au moins un cycle thiazolium est la thiamine ou l'un de ses sels de formule suivante :

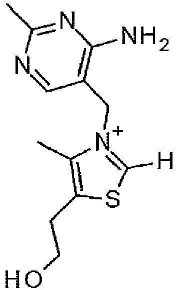

Le composé organique comportant au moins un cycle 1,2,4-triazolium est un composé de formule B suivante : 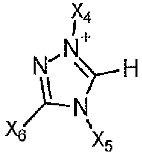 dans laquelle :
X₄ représente un groupement alkyle en C₁ à C₆, aryle en C₅ à C₁₀, notamment phényle, biphényle ou naphthyle, alkyle en C₁ à C₆ aryle en C₅ à C₁₀, notamment benzyle, hétéroaryle en C₅ à C₁₀, notamment pyridyle ou pyrimidyle, lesdits groupements alkyle, aryle, alkyl-aryle et hétéroaryle pouvant être substitués par un ou plusieurs hétéroatomes, notamment choisi parmi N, O ou S,
X₅ représente hydrogène, un groupement alkyle en C₁ à C₆, notamment méthyle, éthyle ou propyle, aryle en C₅ à C₁₀, notamment phényle, biphényle ou naphthyle, alkyle en C₁ à C₆ aryle en C₅ à C₁₀, notamment benzyle, hétéroaryle en C₅ à C₁₀, notamment pyridyle ou pyrimidyle, lesdits groupements alkyle, aryle, alkyl-aryle et hétéroaryle pouvant être substitués par un ou plusieurs hétéroatomes, notamment choisi parmi N, O ou S,
X₆ représente un groupement alkyle en C₁ à C₆, aryle en C₅ à C₁₀, notamment phényle, biphényle ou naphthyle, alkyle en C₁ à C₆ aryle en C₅ à C₁₀, notamment benzyle, hétéroaryle en C₅ à C₁₀, notamment pyridyle ou pyrimidyle, lesdits groupements alkyle, aryle, alkyl-aryle et hétéroaryle pouvant être substitués par un ou plusieurs hétéroatomes, notamment choisi parmi N, O ou S,
ou X₅ et X₆ sont liés entre eux pour former un cycle à 5 ou 6 chaînons, notamment à 5 chaînons, ledit cycle étant éventuellement substitué par un groupement alkyle, aryle, hétéroaryle ou alkyl-aryle. Ledit cycle à 5 ou 6 chaînons substitué par un groupement alkyle, aryle, hétéroaryle ou alkyl-aryle peut être sous la forme d'un mélange d'étantiomère ou être un seul énantiomère.

La présente invention a également pour objet un procédé de préparation d'un produit de formule I : 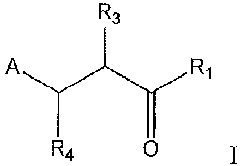 dans laquelle A représente un radical choisi parmi :
- CO-R₂
- CO-O-R₂a
- CN
- C(O) - N Ra R'a
- CH- (CO₂Rb)₂

R₁ représente un atome d'hydrogène ou un radical alkyle ou alkylène linéaire ou ramifié ayant au plus 12 atomes de carbone soit R₁ représente un radical cycloalkyle saturé ou insaturé ayant de 3 à 7 atomes de carbone et comportant éventuellement un ou plusieurs hétéroatomes choisis parmi les atomes d'azote, de soufre ou d'oxygène, soit R₁ représente un radical aryle carbocyclique ou hétérocyclique, chacun de ces radicaux alkyle, alkylène, aryle ou cycloalkyle étant éventuellement substitués,
R₂ représente un radical alkyle linéaire ou ramifié ayant de 1 à 12 atomes de carbone éventuellement substitué soit R₂ représente un radical cycloalkyle saturé ou insaturé ayant de 3 à 7 atomes de carbone et comportant éventuellement un ou plusieurs hétéroatomes choisis parmi les atomes d'azote, de soufre ou d'oxygène soit R₂ représente un radical aryle carbocyclique ou hétérocyclique chacun de ces radicaux alkyle, aryle ou cycloalkyle étant éventuellement substitués,
R₂a représente un radical alkyle linéaire ou ramifié ayant de 1 à 12 atomes de carbone
Ra et R'a identiques ou différents sont choisis parmi radicaux alkyle ou allcoxy linéaires ou ramifiés ayant de 1 à 12 atomes de carbone, étant entendu que Ra et R'a ne peuvent pas représenter simultanément chacun un radical alkoxy linéaire ou ramifié ayant de 1 à 12 atomes de carbone
Rb est choisi parmi les radicaux alkyles linéaires ou ramifiés ayant de 1 à 12 atomes de carbone
R₃ représente un atome d'hydrogène, alkyle ou alkylène linéaire ou ramifié ayant au plus 6 atomes de carbone soit R₃ représente un radical aryle carbocyclique ou hétérocyclique, chacun de ces radicaux alkyle, alkylène ou aryle étant éventuellement substitués,
ou les radicaux R₂ et R₃ sont liés entre eux pour former un cycle ayant de 5 à 7 atomes de carbone, ladite chaine comportant éventuellement un ou plusieurs hétéroatomes choisis parmi les atomes d'azote, de soufre ou d'oxygène,
R₄ est un atome d'hydrogène ou est choisi parmi les radicaux alkyle éventuellement substitués, acylamido ayant de 2 à 12 atomes de carbone, carboxylester, de préférence R₄ représente un atome d'hydrogène,
caractérisé en ce que l'on fait agir un produit de formule II : 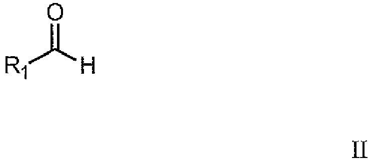 avec un produit de formule III : 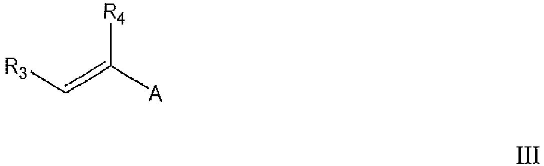
en présence d'une part de la thiamine ou d'un sel de thiamine et d'autre part d'acide ascorbique sous forme libre ou salifiée ou d'un sel d'acide organique de métal alcalin de préférence l'acétate de sodium, le tartrate de potassium, le succinate de sodium, ou d'un composé comprenant un motif 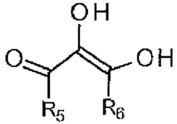
dans lequel R₅ et R₆ représentent indépendamment l'un de l'autre un atome d'hydrogène, un radical alkyle linéaire ou ramifié ayant de 1 à 12 atomes de carbone ou R₅ et R₆ sont liés entre eux pour former un cycle ayant de 3 à 7 chaînons, ledit alkyle ou ledit cycle étant éventuellement substitué par un ou plusieurs hétéroatomes, notamment choisi parmi O, N et S, notamment une réductone, de préférence le 2-hydroxypropanedial ou la 2,3-dihydroxycyclopent-2-en- 1-one ou l'acide croconique, dans un solvant organique.

Dans ce qui précède, la réaction est mise en oeuvre en présence simultanée lors de la réaction entre les produits II et III de la thiamine ou d'un sel de thiamine et de l'un (ou plusieurs) des produits choisis parmi l'acide ascorbique sous forme libre ou salifiée ou d'un composé comprenant un motif 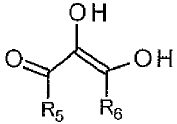 dans lequel R₅ et R₆ représentent indépendamment l'un de l'autre un atome d'hydrogène, un radical alkyle linéaire ou ramifié ayant de 1 à 12 atomes de carbone ou R₅ et R₆ sont liés entre eux pour former un cycle ayant de 3 à 7 chainons, ledit alkyle ou ledit cycle étant éventuellement substitué par un ou plusieurs hétéroatomes, notamment choisi parmi O, N et S, notamment une réductone, de préférence le 2-hydroxypropanedial ou la 2,3-dihydroxycyclopent-2-en- 1-one ou l'acide croconique.

La présente invention a également pour autre objet un procédé de préparation d'un produit de formule I : 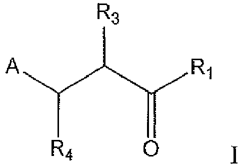 dans laquelle A représente un radical choisi parmi :
- CO-R₂
- CO-O-R₂a
- CN
- C(O) - N Ra R'a
- CH- (CO₂Rb)₂

R₁ représente un atome d'hydrogène ou un radical alkyle ou alkylène linéaire ou ramifié ayant au plus 12 atomes de carbone soit R₁ représente un radical cycloalkyle saturé ou insaturé ayant de 3 à 7 atomes de carbone et comportant éventuellement un ou plusieurs hétéroatomes choisis parmi les atomes d'azote, de soufre ou d'oxygène, soit R₁ représente un radical aryle carbocyclique ou hétérocyclique, chacun de ces radicaux alkyle, alkylène, aryle ou cycloalkyle étant éventuellement substitués,
R₂ représente un radical alkyle linéaire ou ramifié ayant de 1 à 12 atomes de carbone éventuellement substitué soit R₂ représente un radical cycloalkyle saturé ou insaturé ayant de 3 à 7 atomes de carbone et comportant éventuellement un ou plusieurs hétéroatomes choisis parmi les atomes d'azote, de soufre ou d'oxygène soit R₂ représente un radical aryle carbocyclique ou hétérocyclique chacun de ces radicaux alkyle, aryle ou cycloalkyle étant éventuellement substitués,
R₂a représente un radical alkyle linéaire ou ramifié ayant de 1 à 12 atomes de carbone Ra et R'a identiques ou différents sont choisis parmi radicaux alkyle ou alkoxy linéaires ou ramifiés ayant de 1 à 12 atomes de carbone, étant entendu que Ra et R'a ne peuvent pas représenter simultanément chacun un radical alkoxy linéaire ou ramifié ayant de 1 à 12 atomes de carbone
Rb est choisi parmi les radicaux alkyles linéaires ou ramifiés ayant de 1 à 12 atomes de carbone
R₃ représente un atome d'hydrogène, alkyle ou alkylène linéaire ou ramifié ayant au plus 6 atomes de carbone soit R₃ représente un radical aryle carbocyclique ou hétérocyclique, chacun de ces radicaux alkyle, alkylène ou aryle étant éventuellement substitués,
R₄ est un atome d'hydrogène ou est choisi parmi les radicaux alkyle éventuellement substitués, acylamido ayant de 2 à 12 atomes de carbone, carboxylester, de préférence R₄ représente un atome d'hydrogène,
caractérisé en ce que l'on fait agir un produit de formule II : 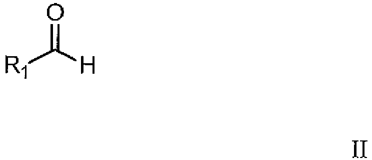 avec un produit de formule III : 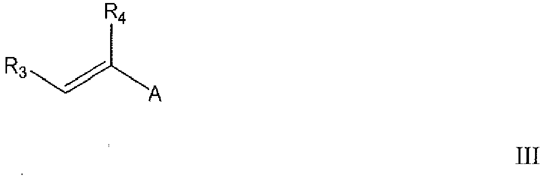
en présence d'une part de la thiamine ou d'un sel de thiamine et d'autre part d'acide ascorbique sous forme libre ou salifiée ou d'un sel d'acide organique de métal alcalin de préférence l'acétate de sodium, le tartrate de potassium, le succinate de sodium, d'un composé comprenant un motif 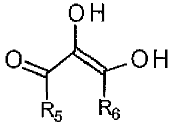
dans lequel R₅ et R₆ représentent indépendamment l'un de l'autre un atome d'hydrogène, un radical alkyle linéaire ou ramifié ayant de 1 à 12 atomes de carbone ou R₅ et R₆ sont liés entre eux pour former un cycle ayant de 3 à 7 chaînons, ledit alkyle ou ledit cycle étant éventuellement substitué par un ou plusieurs hétéroatomes, notamment choisi parmi O, N et S, notamment une réductone, de préférence le 2-hydroxypropanedial ou la 2,3-dihydroxycyclopent-2-en- 1-one ou l'acide croconique dans un solvant organique.

Dans ce qui précède, la réaction est mise en oeuvre en présence simultanée lors de la réaction entre les produits II et III de la thiamine ou d'un sel de thiamine et de l'un (ou plusieurs) des produits choisis parmi l'acide ascorbique sous forme libre ou salifiée ou d'un sel d'acide organique de métal alcalin de préférence l'acétate de sodium, le tartrate de potassium, le succinate de sodium, d'un composé comprenant un motif 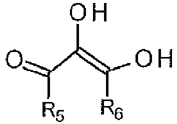 dans lequel R₅ et R₆ représentent indépendamment l'un de l'autre un atome d'hydrogène, un radical alkyle linéaire ou ramifié ayant de 1 à 12 atomes de carbone ou R₅ et R₆ sont liés entre eux pour former un cycle ayant de 3 à 7 chaînons, ledit alkyle ou ledit cycle étant éventuellement substitué par un ou plusieurs hétéroatomes, notamment choisi parmi O, N et S, notamment une réductone, de préférence le 2-hydroxypropanedial ou la 2,3-dihydroxycyclopent-2-en- 1-one ou l'acide croconique.

De préférence, la réaction est mise en oeuvre en présence simultanée lors de la réaction entre les produits II et III de la thiamine ou d'un sel de thiamine et de l'un (ou plusieurs) des produits choisis parmi l'acide ascorbique sous forme libre ou salifiée ou d'un composé comprenant un motif 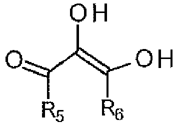 dans lequel R₅ et R₆ représentent indépendamment l'un de l'autre un atome d'hydrogène, un radical alkyle linéaire ou ramifié ayant de 1 à 12 atomes de carbone ou R₅ et R₆ sont liés entre eux pour former un cycle ayant de 3 à 7 chainons, ledit alkyle ou ledit cycle étant éventuellement substitué par un ou plusieurs hétéroatomes, notamment choisi parmi O, N et S, notamment une réductone, de préférence le 2-hydroxypropanedial ou la 2,3-dihydroxycyclopent-2-en- 1-one ou l'acide croconique.

Le réactif aldéhydique R₁CHO peut être un aldéhyde aliphatique, aromatique carbocyclique ou hétérocyclique, mais aussi le formaldéhyde (R₁ = H) ce qui ne conduit pas au produit I dans le cas de la réaction de Stetter conventionnelle.

Les radicaux alkyle que peuvent représenter R₁, R₂, R₃ et R₄ peuvent de préférence être fonctionnalisés par un autre motif par exemple un radical carbonyle (cétone ou aldéhyde) libre ou protégé sous forme de cétal, un carboxy sous forme libre ou sous forme d'ester carboxylique, ou être substitué par un aryle hétérocyclique.

Comme indiqué ci-dessus, les réductones préférées sont le 2-hydroxypropanedial ou la 2,3-dihydroxycyclopent-2-en- 1-one. Ces produits possèdent également d'autres noms, par exemple, respectivement le tartronaldéhyde ou hydroxypropanedial et acide réductique.

Leurs formules développées sont indiquées ci-dessous :

| **Exemples de reductones** | | |
|---|---|---|
| 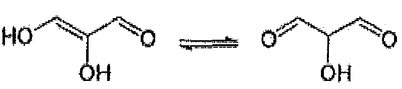 | 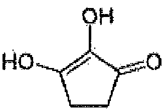 | 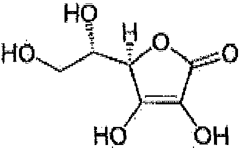 |
| **Tartronaldéhyde ou hydroxypropanedial** | **Acide réductique** | **Acide ascorbique (Vitamine C)** |

L'acide croconique a quant à lui la formule suivante :

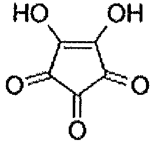

L'invention a plus spécialement pour objet un procédé tel que décrit ci-dessus caractérisé en ce que le ou les substituants que peuvent porter les radicaux alkyle ou alkylène linéaire ou ramifié, aryle carbocyclique ou hétérocyclique ou cycloalkyle sont choisis parmi les radicaux aryle carbocyclique ou hétérocyclique, eux-mêmes éventuellement substitués, les radicaux carboxylique libre estérifié ou salifié, le groupement oxo libre sous forme de cétone ou protégé sous forme de cétal, les atomes d'halogène, les radicaux alkoxy ayant de 1 à 6 atomes de carbone tel que méthoxy ou éthoxy.

R₁ et R₂ peuvent représenter un radical alkyl ou alkényle ayant de préférence au plus 9 atomes de carbone et plus préférentiellement encore au plus 7 atomes de carbone.

Les radicaux alkyles peuvent de préférence être substitués par un radical oxo libre sous forme de cétone ou protégé sous forme de cétal.

Les radicaux aryle carbocycliques sont choisis de préférence parmi les radicaux phényle éventuellement mono-, di- ou tri-substitué par un ou plusieurs radicaux choisis parmi les alkyle ou alcoxy ayant de 1 à 6 atomes de carbone, un radical hydroxy, un atome d'halogène, un radical cyano, trifluorométhyle, alkylènedioxy ayant de 1 à 6 atomes de carbone, ou le radical naphtyle,
Les radicaux aryle hétérocycliques monocycliques ou condensés sont choisis de préférence parmi les radicaux pyridyle, quinoléinyle, isoquinoléinyle, benzothiényle, benzofuranyle, benzopyranyle, benzothiopyranyle, furanyle, pyrrolyle, thiazolyle, oxazolyle, isoxazolyle, triazolyle, tétrazolyle, pyrazolyle, imidazolyle, thiényle, phtalimide ou l'un quelconque desdits radicaux substitué par un alkyle ou un alcoxy ayant de 1 à 12 atomes de carbone ou un halogène.

Les radicaux radical cycloalkyle saturés ou insaturés mono ou bicycliques ayant de 3 à 7 atomes de carbone et comportant éventuellement un ou plusieurs hétéroatomes choisis parmi les atomes d'azote, de soufre ou d'oxygène sont choisis de préférence parmi les radicaux cyclopropyle, cyclobutyle, cyclopentyle, cyclohexyle, cycloheptyle, cyclooctyle, cyclopropènyle, cyclobutènyle, cyclopentènyle, cyclohexènyle, cycloheptènyle, 1,3-cyclohexadiènyle, 1,4-cyclohexadiènyle, 1,5-cyclohexadiènyle, bicycloheptényle
Le groupe des hétérocycloalkyles mono ou bicycliques c'est-à-dire les cycloallcyles comportant un ou plusieurs hétéroatomes choisis parmi les atomes d'azote, de soufre ou d'oxygène peut inclure les radicaux suivants : aziridinyle, pyrrolidinyle, pyrrolidino, pipéridinyle, pipérazinyle, pipérazino, morpholinyle, thiomorpholinyle, tétrahydrofuranyle, tétrahydrothiofuranyle, tétrahydropyranyle, dihydropyranyle, dioxanyle, dioxolanyle et pyranyle.

Le radical carboxylique qui peut être estérifié par un radical alkyle ayant de 1 à 4 atomes de carbone tel qu'un radical méthyle, éthyle ou tert-butyle, le radical alkyle peut être substitué par un ou plusieurs atomes d'halogène tel que le fluor, le chlore ou le brome tel que le trifluorométhyle.

Les atomes d'halogène sont choisis parmi les atomes de fluor, de chlore de brome ou d'iode,
Les radicaux alcoxy ont de 1 à 12 atomes de carbone et de préférence de 1 à 7 atomes de carbone. Les radicaux préférés sont les radicaux méthoxy, éthoxy, propyloxy, isopropyloxy, n-butoxy ou tert-butyloxy,
R₁, R₂ et R₃ peuvent également représenter l'un des radicaux aryle carbocycliques ou hétérocycliques éventuellement substitués cités précédemment ;
Les valeurs de R₁ R₂ R₃ et R₄ choisies parmi les valeurs indiquées précédemment peuvent être identiques ou différentes.

De préférence R₁ est choisi parmi un atome d'hydrogène ou un radical alkyle linéaire ou ramifié ayant de 1 à 9 atomes de carbone, de préférence méthyle, éthyle, n-propyle, le butyle, l'isobutyle, le tert-butyle, l'hexyle, l'octyle, le nonyle ou un radical alkylène linéaire ou ramifié ayant au plus 9 atomes de carbone de préférence le 1-pentényle, le 1-hexényle, le 2,6-di méthyl 5-heptényle, le 2,6-di méthyl -1,5 diényl heptyle, le 1-nonényle, un radical benzyle ou un radical phényle, un hétérocycle aromatique ou non aromatique oxygéné ou azoté, et plus préférentiellement le n-propyle, le butyle, l'isobutyle, ou le tert-butyle l'hexyle, l'octyle, le nonyle ou un radical alkylène linéaire ou ramifié ayant au plus 9 atomes de carbone de préférence le 1-pentényle, le 1-hexényle, le 3-hexényle, le 2,6-di méthyl 5-heptényle, le 2,6-di méthyl -1,5 diényl heptyle, le 1-nonényle, un radical benzyle ou un radical phényle, ces radicaux peuvant être substitués comme indiqué ci-dessus.

Les substituants R₂ et R₂a peuvent représenter de préférence un radical alkyle linéaire ou ramifié ayant de 1 à 9 atomes de carbone, de préférence méthyl, éthyl, n-propyle, le butyle, l'isobutyle, le tert-butyle, l'hexyle, l'octyle, le nonyle.

Les substituants Ra et R'a peuvent représenter les mêmes valeurs que celles citées précédemment lorsqu'ils représentent un radical alkyle linéaire ou ramifié ayant de 1 à 9 atomes de carbone, de préférence le n-propyle, l'isobutyle, l'hexyle, l'octyle, le nonyle ou un radical alkylène linéaire ou ramifié ayant au plus 9 atomes de carbone de préférence le 1-pentényle, le 1-hexényle, le 2,6-di méthyl 5-heptényle, le 2,6-di méthyl -1,5 diényl heptyle, le 1-nonényle, Ra et R'a peuvent également représenter un radical benzyle ou un radical phényle.

Ra et R'a peuvent également représenter les valeurs alkoxy correspondantes telles que par exemple méthoxy ou éthoxy.

Rb est choisi parmi les valeurs alkyles citées précédemment et a de préférence de 1 à 7 atomes de carbone.

R₃ peut représenter l'une des valeurs alkyle ou aryle citées précédemment. De préférence, R₃ représente un atome d'hydrogène ou un radical alkyle, de préférence un radical méthyle, substitué par un radical oxo libre sous forme de cétone ou protégé sous forme de cétal, un diol protégé sous forme de cétal, ou un radical carboxylique sous forme libre, salifiée ou estérifiée par exemple par un radical alkyle ayant de 1 à 4 atomes de carbone ou par un radical aryle carbocyclique ou hétérocyclique. Plus préférentiellement encore R₃ représente un atome d'hydrogène.

La valeur acylamido que peut représenter R₄ est de préférence la valeur acétylamido.

R₄ peut également représenter de préférence un atome d'hydrogène un radical alkyle, de préférence un radical méthyle substitué par un radical oxo libre sous forme de cétone ou protégé sous forme de cétal ou un radical carboxylique sous forme libre, salifiée ou estérifiée par exemple par un radical alkyle ayant de 1 à 4 atomes de carbone.

Lorsque les radicaux R₂ et R₃ sont liés entre eux pour former un cycle ayant de 5 à 7 atomes de carbone, ledit cycle est de préférence une cyclopenténone.

La présente invention a plus particulièrement pour objet un procédé se rapportant au procédé décrit ci-dessus de préparation d'un produit de formule Ia 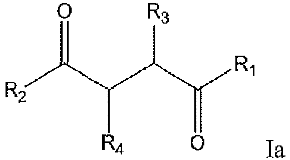 dans laquelle R₁, R₃ et R₄ ont la signification indiquée ci-dessus et correspondant à un produit de formule I telle que définie ci-dessus dans laquelle A représente un radical CO-R₂ dans lequel R₂ a la signification indiquée précédemment
caractérisé en ce que l'on fait agir un produit de formule II tel que défini ci-dessus avec un produit de formule IIIa : 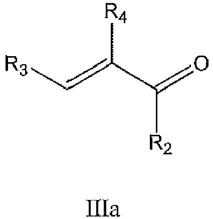 en présence de thiamine ou d'un sel de thiamine de préférence le chlorhydrate de thiamine et d'acide ascorbique sous forme libre ou salifiée de préférence l'ascorbate de sodium dans un solvant organique, de préférence l'éthanol.

La réaction est conduite à une température de 50 à 120 °C, avantageusement de 70 à 90 °C.

Le solvant dans lequel est mise en oeuvre la réaction est avantageusement un alcool, notamment choisi parmi le méthanol, l'éthanol, le 1-propanol, le 2-propanol, le butanol, un polyol, notamment un diol tel que le 1,2- propanediol ou le 1,3- propanediol ou un triol tel que le glycérol. Le solvant peut également être un mélange de ces alcools ou polyols, un mélange d'un alcool ou d'un polyol avec un solvant organique ou un mélange d'un alcool ou d'un polyol et d'eau. Il s'agit de préférence de l'éthanol, du 1-propanol, du 2-propanol, du 1,2- propanediol, du 1,3- propanediol, du glycérol et de l'eau ou d'un mélange de deux ou plus de ces solvants. De manière préférée, le solvant est un diol ou un triol, notamment le 1,2-propanediol, le 1,3- propanediol, le glycérol ou un mélange de deux ou trois de ces solvants.

La réaction est conduite de préférence sous courant de diazote, pendant une durée de 30 minutes à 30 heures, notamment de l'ordre de 1 à 12 h, de préférence de l'ordre de 2 heures.

La quantité de solvant est avantageusement de 1 à 5 L, notamment d'environ 2,5 litres de solvant par mole de produit IIIa. On utilise de préférence de l'ordre de 1 à 5 litres d'éthanol par mole de produit IIIa.

On utilise de préférence un excès de produit de formule II par rapport au produit de formule III ou IIIa. Cet excès peut être de l'ordre de 4 / 1 à 1,1 / 1, notamment de 2/1 par exemple entre 1,5 et 2,5, de préférence 2. On utilise environ 0,1 mole de thiamine ou d'un sel de thiamine pour 1 mole de produit de formule II et de 0,2 à 1,4 moles, notamment de 0,2 à 0,8, de préférence de l'ordre de 0,6 mole, d'acide ascorbique sous forme libre ou salifiée pour 1 mole de produit de formule II. Le ratio thiamine/acide ascorbique sous forme libre ou salifiée varie de 1/2 à 1/20, avantageusement de 1/2 à 1/10, plus avantageusement de 1/3 à 1/7, et est de préférence 1/5 ou 1/6.

Le procédé peut être mis en oeuvre en mélangeant ensemble l'acide ascorbique sous forme libre ou salifiée et le sel de thiamine puis en ajoutant à ce mélange le produit de formule II et le produit de formule III ou IIIa. Avantageusement, l'acide ascorbique sous forme libre ou salifiée et le sel de thiamine sont agités dans le solvant pendant une durée de 1 à 30 minutes, notamment d'environ 10 minutes avant l'ajout des produits de formule II et III ou IIIa.

Le procédé peut également être mis en oeuvre en ajoutant le mélange d'acide ascorbique sous forme libre ou salifiée et de sel de thiamine au mélange de produits de formule II et III ou IIIa. Dans un mode de réalisation avantageux, le mélange d'acide ascorbique sous forme libre ou salifiée et de sel de thiamine est ajouté par portions. Dans un autre mode de réalisation avantageux, le sel de thiamine est ajouté par portions à un mélange de produits de formule II et III ou IIIa et d'acide ascorbique sous forme libre ou salifiée.

Par « ajouté par portions », on entend au sens de la présente invention que la quantité totale d'acide ascorbique sous forme libre ou salifiée et de sel de thiamine est ajoutée en plusieurs fois au cours de la réaction. Selon un mode de réalisation avantageux, l'acide ascorbique sous forme libre ou salifiée et le sel de thiamine sont ajoutés par portions représentant 25 % puis 25 % puis 25 % puis 25 % de la quantité totale d'acide ascorbique sous forme libre ou salifiée et de sel de thiamine.

Dans un autre mode de réalisation préféré, le sel de thiamine est ajouté en quatre portions de 25 % chacune au mélange de produits de formule II et III ou IIIa et d'acide ascorbique sous forme libre ou salifiée.

Le schéma suivant illustre le procédé de l'invention :

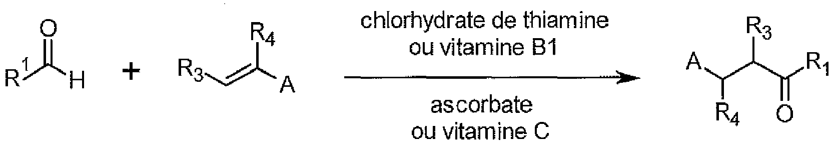

L'accepteur de Michael : 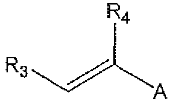 peut être une cétone (A = - CO-R₂), un ester, (A = -CO-O-R₂a), un nitrile α,β-éthylénique (A = -CN).

Il peut porter d'autres groupes fonctionnels tels qu'un cétal, un carboxy ester ou un bicycloheptényle.

Les Inventeurs ont également mis en évidence que dans les conditions réactionnelles décrites ci-dessus, en présence d'une part d'un sel de thiazolium, d'un sel d'1,3-imidazolium ou d'un sel de 1,2,4-triazolium, tel que défini ci-dessus et d'autre part d'acide ascorbique sous forme libre ou salifiée, ou d'un composé comprenant un motif 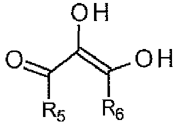 dans lequel R₅ et R₆ représentent indépendamment l'un de l'autre un atome d'hydrogène, un radical alkyle linéaire ou ramifié ayant de 1 à 12 atomes de carbone ou R₅ et R₆ sont liés entre eux pour former un cycle ayant de 3 à 7 chainons, ledit alkyle ou ledit cycle étant éventuellement substitué par un ou plusieurs hétéroatomes, notamment choisi parmi O, N et S, notamment une réductone, de préférence le 2-hydroxypropanedial ou la 2,3-dihydroxycyclopent-2-en- 1-one ou l'acide croconique dans un solvant organique, mais en l'absence d'un composé de formule III ou IIIa, une auto-condensation de l'aldéhyde était observée (condensation acyloine).

La présente invention concerne donc également un procédé de préparation d'un composé de formule III' : 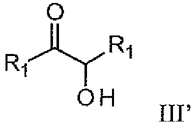 caractérisé en ce que l'on fait agir un produit de formule II : 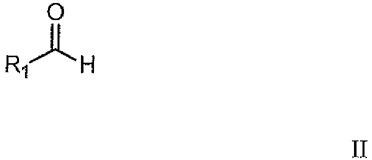 dans laquelle :
R₁ représente un radical alkyle ou alkylène linéaire ou ramifié ayant au plus 12 atomes de carbone soit R₁ représente un radical cycloalkyle saturé ou insaturé, ayant de 3 à 7 atomes de carbone et comportant éventuellement un ou plusieurs hétéroatomes choisis parmi les atomes d'azote, de soufre ou d'oxygène, soit R₁ représente un radical aryle carbocyclique ou hétérocyclique, chacun de ces radicaux alkyle, alkylène, aryle ou cycloalkyle étant éventuellement substitués,
en présence d'une part d'un sel de thiazolium, d'un sel d'1,3-imidazolium ou d'un sel de 1,2,4-triazolium, tel que défini ci-dessus et d'autre part d'acide ascorbique sous forme libre ou salifiée, ou d'un composé comprenant un motif 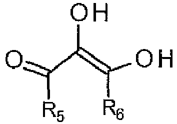
dans lequel R₅ et R₆ représentent indépendamment l'un de l'autre un atome d'hydrogène, un radical alkyle linéaire ou ramifié ayant de 1 à 12 atomes de carbone ou R₅ et R₆ sont liés entre eux pour former un cycle ayant de 3 à 7 chaînons, ledit alkyle ou ledit cycle étant éventuellement substitué par un ou plusieurs hétéroatomes, notamment choisi parmi O, N et S, notamment une réductone, de préférence le 2-hydroxypropanedial ou la 2,3-dihydroxycyclopent-2-en- 1-one ou l'acide croconique dans un solvant organique, mais en l'absence d'un composé de formule III ou IIIa.

De préférence, lorsque le radical R₁ est alkylène ou un radical cycloalkyle insaturé, la ou es doubles liaisons ne sont pas conjuguées à la fonction aldéhyde.

De préférence, la réaction est mise en oeuvre dans les conditions décrites ci-dessus, et de manière particulièrement préférée en présence de vitamine B1 et d'acide ascorbique sous forme libre ou salifiée.

Parmi les différentes adduits de Michael issus de la réaction de Stetter, il est connu que les dérivés dicarbonylés 1,4 sont les précurseurs directs de nombreux hétérocycles à 5 chaînons. Ils présentent spécialement un intérêt particulier dans le domaine des dérivés cosmétiques. Ils constituent des précurseurs naturels des cyclopenténones, et donc des dérivés jasmoniques. Ainsi par exemple, en utilisant les catalyseurs décrits dans la demande PCT/FR2014/052280 du 12 septembre 2014 publiée sous le numéro WO 2015/036714, les inventeurs ont montré qu'il était possible de préparer la dihydrojasmone à l'aide de catalyseurs basiques biosourcés:

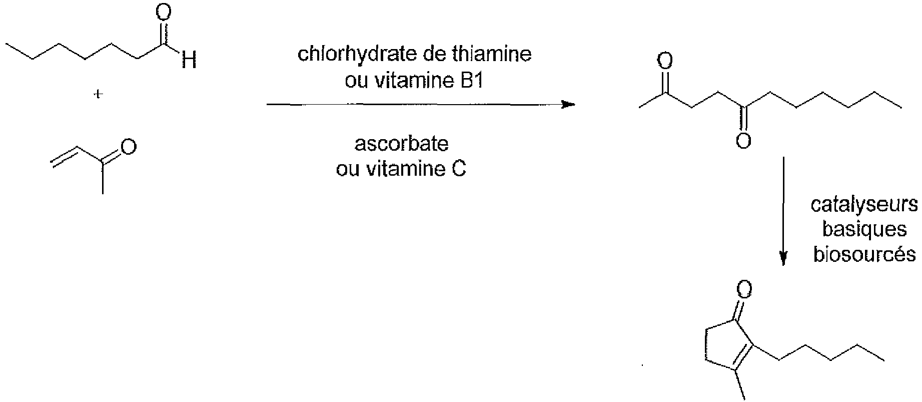

On décrit aussi un procédé de préparation d'un produit de formule IV' : 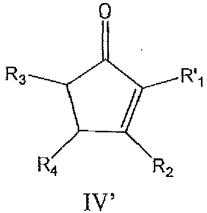 dans laquelle R'₁ représente un atome d'hydrogène ou un radical alkyle ayant au plus 11 atomes de carbone éventuellement substitué, R₂ et R₃ ont la signification indiquée ci-dessus, étant entendu que si R₃ et R₄ représentent chacun un atome d'hydrogène et R'₁ un radical pentyle, alors R₂ ne peut pas représenter un radical méthyle caractérisé en ce que l'on fait agir un produit de formule l'a : 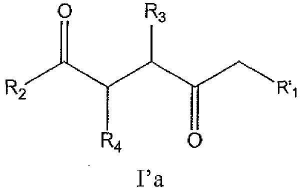 avec un catalyseur métallique, de préférence un catalyseur obtenu à partir de matériaux d'origine organique contenant des métaux alcalins ou alcalino-terreux, de préférence le calcium.

La réaction de cyclisation conduisant à un cycle penténone peut également être effectuée dans le cas où R₂ représente un atome d'hydrogène.

On décrit aussi un procédé de préparation d'un produit de formule IV" : 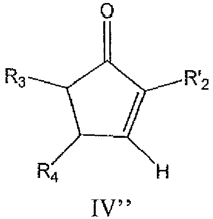 dans laquelle R'₂ représente un atome d'hydrogène ou un radical alkyle ayant au plus 11 atomes de carbone éventuellement substitué, R₂ et R₃ ont la signification indiquée ci-dessus caractérisé en ce que l'on fait agir un produit de formule I"a 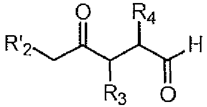 I"a avec un catalyseur métallique, de préférence un catalyseur obtenu à partir de matériaux d'origine organique contenant des métaux alcalins ou alcalino-terreux, de préférence le calcium.

Les radicaux R'₁ et R'₂ peuvent porter les mêmes substituants que les radicaux R₁ et R₂.

Les matériaux d'origine organique contenant des métaux alcalins ou alcalino-terreux, de préférence le calcium utilisables comme catalyseur basique dans les réactions de cyclisation indiquées ci-dessus sont de préférence constitués d'un extrait d'une plante ou d'une partie de plante d'une algue ou d'une partie d'algue comportant un taux important de calcium (Ca), en quantité de préférence supérieure à 50000 ppm en poids, et contenant moins de 5000 ppm de métaux choisis parmi le Zinc (Zn), le Nickel (Ni), le Manganèse (Mn) le Plomb (Pb), le cadmium (Cd), le cuivre (Cu), le palladium (Pd) ou de coquilles marines ou de coquilles de mollusques non marins contenant un taux important de métal alcalin ou alcalino-terreux, de préférence le calcium (Ca), de préférence sous forme de carbonate de calcium, en quantité de préférence supérieure à 80%, plus préférentiellement supérieure à 90% en poids, éventuellement après séchage et/ou broyage et traitement thermique et/ou chimique ledit catalyseur étant sous forme d'oxyde de calcium, de sel de calcium choisi parmi l'oxalate, le carbonate, les phosphates tels que le phytate, les carboxylates simples tels que le citrate, ou les carboxylates sodiques tels que les uronates de type alginates ou polygalacturonates ou d'hydroxyde de calcium.

La plante comportant un taux important de calcium (Ca), en quantité de préférence supérieure à 50000 ppm en poids, sous forme de sel tel que l'oxalate de calcium ou le carbonate de calcium est une plante choisie de préférence parmi :
- les Chénopodiacées, de préférence le chénopode blanc
- les Plantaginacées de préférence le grand plantain
- les Portulacacées le pourpier commun
- les algues calcifiantes de préférence le Lithothamnium.

Les catalyseurs peuvent être préparés comme suit :
- **CAT A1** : 420g d'échantillons déshydratés de plantes riches en Ca, de préférence le plantain sont brûlés dans un four à moufle à 500°C pendant 7h. 183 g d'un solide riche en carbonates métalliques sont obtenus.
- **CAT A1 supporté** : 1,7g d'un solide riche en carbonates métalliques sont co-broyés avec 5g de support (ex : alumine basique, Montmorillonite), puis activés par 15 minutes de chauffage à 150°C.

Le solide riche en carbonate comprend aussi d'autres anions, notamment des phosphates.
- **CAT A2 :** 10 g de carbonates métalliques issus du traitement thermique précédent sont introduits dans un bécher et arrosés d'eau sous agitation. 30 mL d'eau sont ajoutés. Une suspension grise est obtenue ; l'agitation est maintenue 1h.

Après décantation, le pH est de 10. Le milieu est concentré à l'évaporateur rotatif, séché à l'étuve à 80°C. 10, 1 g d'une poudre grise sont obtenus.
- **CAT A2 supporté** : 1,7g de carbonates métalliques sont co-broyés avec 5g de support (ex : alumine basique), puis activés par 15 minutes de chauffage à 150°C.
- **CAT A3:** 10g de **CAT A1** sont introduites dans un ballon de 250 mL avec 50 mL d'HCl 12M sont ajoutés sous agitation. La solution obtenue est filtrée sur célite. Après évaporation et concentration, 4,9g d'un solide est recueilli et séché à 80°C. 3 g du solide précédent sont dissous dans 70 mL d'eau distillée avec quelques gouttes d'HCl pour favoriser la dissolution totale. La précipitation des hydroxydes métalliques est réalisée par ajout d'une solution de soude concentrée jusqu'à pH = 13. La suspension blanche obtenue. Elle est centrifugée. 2,7 g de solide sont obtenus et conservés à l'étuve.
- **CAT A5** : le catalyseur dérivé des coquilles d'huitres est préparé comme suit :
   Les coquilles sont directement placées dans un four à calciner. Celui-ci est porté à 1000°c pendant environ 7h. La poudre résultante, très riche en oxyde calcium, est conservée sous atmosphère inerte, ou utilisée directement. Dans ce cas, elle est réhydratée par ajouts progressifs dans l'eau. Le pH est alors d'environ 12. La phase aqueuse obtenue peut constituer le milieu réactionnel ou subir la séquence filtration / séchage à l'étuve. Dans ce cas, le solide obtenu peut être utilisé comme catalyseur ou réactif dans un autre milieu, tel qu'un milieu éthanolique.

Les conditions opératoires dans lesquelles la réaction de cyclisation est effectuée peuvent être décrites comme suit :
0,5 M de produit de formule l'a ou I"a est diluée dans un mélange d'eau et d'éthanol (80/20 mL). 500 mg d'hydroxydes de plantain préparés par hydrolyse alcaline des chlorures correspondants (CAT A3) ou à partir de CAT A5. La solution est portée à reflux 16h, puis extraite à l'éther. Un suivi de la réaction par GC MS montre la formation complète de la dihydrojasmone. Le catalyseur est recyclé par filtration, lavage à l'eau, à l'éthanol puis l'acétone et séchage 4h à 120°C.

Les inventeurs de la présente demande ont également constaté que, de manière tout à fait inattendue, si le substrat aldéhydique est conjugué à une double liaison éthylénique (exemple : 2-heptenal), le cocktail vitamine C/vitamine B1 peut conduire soit à la dicétone insaturéee telle que décrite précédemment, soit directement à la cyclopenténone correspondante sans que l'ajout d'une base supplémentaire ou d'un catalyseur de type basique ne soit nécessaire, contrairement aux conditions de Stetter, qui conduisent à une cyclisation du composé 1,4-dicarbonylé après ajout d'une base telle que NaOH.

De manière générale, lorsque le substrat aldéhydique est conjugué à une double liaison éthylénique la cyclisation conduisant à la cyclopenténone se produit spontanément au moins en partie dans les conditions de la réaction de Stetter telle que modifiée par les inventeurs de la présente invention (cocktail vitamine C/vitamine B1 de préférence).

Il a de plus été constaté de façon inattendue que le pourcentage de cyclisation augmente lorsqu'un excès de vitamine C (acide ascorbique ou sel de l'acide ascorbique) est introduit dans le milieu réactionnel.

Dans le cas de cette méthode, en ajustant comme indiqué ci-dessus les conditions réactionnelles, la cyclisation peut devenir spontanée sur la totalité ou la quasi-totalité de l'intermédiaire 1,4-dicarbonylé insaturé, formé à partir d'un énal.

La présente invention a donc également pour objet un de préparation d'un produit de formule IVi : 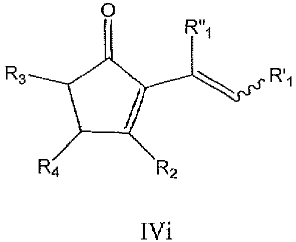 dans laquelle R'₁ représente un radical alkyle ou alkylène linéaire ou ramifié ayant au plus 12 atomes de carbone, éventuellement substitué soit R'₁ représente un radical aryle carbocyclique ou hétérocyclique, R"₁ représente un atome d'hydrogène ou radical alkyle ayant au plus 6 atomes de carbone
caractérisé en ce que l'on fait agir un produit de formule IIi 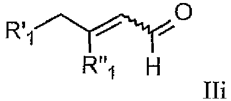 avec un produit de formule IIIa : 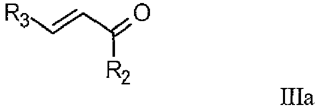 tel que défini ci-dessus en présence de thiamine ou d'un sel de thiamine, de préférence le chlorhydrate de thiamine et d'acide ascorbique sous forme libre ou salifiée de préférence l'ascorbate de sodium dans un solvant organique pour obtenir un produit de formule Ii : 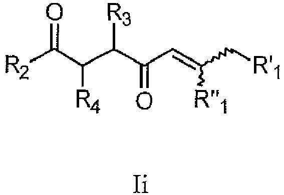 produit de formule Ii qui se transforme spontanément en produit de formule IVi en présence de thiamine ou d'un sel de thiamine, de préférence le chlorhydrate de thiamine et d'acide ascorbique sous forme libre ou salifiée de préférence l'ascorbate de sodium, de préférence en présence d'un excès d'ascorbate de sodium.

Sans que cela constitue une limitation à la présente invention, les inventeurs pensent que l'effet conduisant à la cyclisation spontanée du produit de formule I'b est dû uniquement à l'action de la vitamine C ajoutée initialement. Son action totalement inattendue a pu être démontrée par préparation de l'intermédiaire 1,4-dicarbonylé insaturé selon les conditions classiques de Stetter (seul produit obtenu avec Et₃N comme base), puis mise en réaction de cet intermédiaire avec de la vitamine C : la cyclisation se produit à hauteur de 31 % de rendement. :

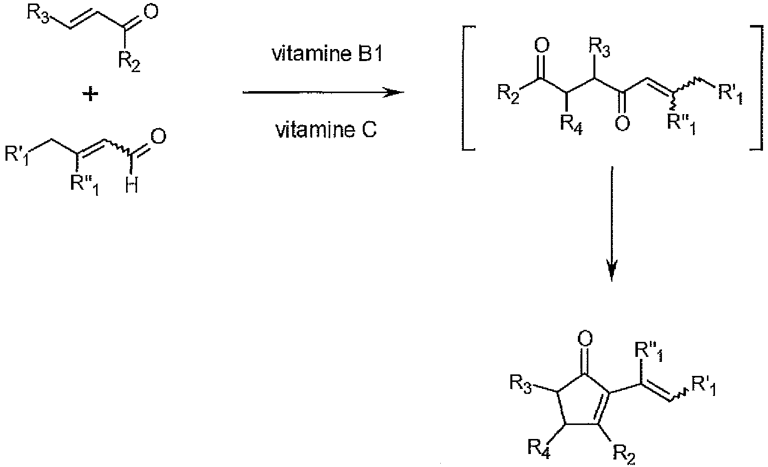

La présente invention concerne également l'utilisation d'un mélange d'un sel de thiazolium, d'un sel d' 1,3-imidazolium ou d'un sel de 1,2,4-triazolium, notamment d'un sel de thiazolium ou d'un sel de 1,2,4-triazolium, de préférence d'un sel de thiazolium et d'acide ascorbique sous forme libre ou salifiée pour la préparation de composés 1,4-dicarbonylés linéaires, de préférence les produits de formules I, Ia, l'a, I"a ou Ii ou de cyclopenténones, tels que la dihydrojasmone et ses dérivés de préférence les produits de formules IV', IV" ou IVi, de préférence dans une réaction de type Stetter.

La présente invention concerne en outre l'utilisation d'un mélange de vitamine B1 (thiamine) ou d'un sel de thiamine et d'acide ascorbique sous forme libre ou salifiée pour la préparation de composés 1,4-dicarbonylés linéaires, de préférence les produits de formules I, Ia, I'a, I"a ou Ii ou de cyclopenténones, tels que la dihydrojasmone et ses dérivés de préférence les produits de formules IV', IV" ou IVi, de préférence dans une réaction de type Stetter.

Avantageusement, le mélange de thiamine ou d'un sel de thiamine et d'acide ascorbique sous forme libre ou salifiée est un mélange comprenant un excès d'acide ascorbique. Le ratio thiamine/acide ascorbique sous forme libre ou salifiée varie de 1/2 à 1/20, avantageusement de 1/2 à 1/10, plus avantageusement de 1/3 à 1/7, et est de préférence 115 ou 116.

### Exemples :

### Exemple 1 - Préparation de dicétones :

### Mode opératoire :

Dans un ballon muni d'un barreau magnétique, d'un réfrigérant et d'une arrivée d'azote, sont introduits 10 mL d'éthanol anhydre, 33,7 mg (0,1 mmol) de chlorhydrate de thiamine et 119 mg (0,6 mmol) d'ascorbate de sodium. L'ensemble est agité à température ambiante durant 10 minutes puis sont ajoutés 140 mg (2,0 mmol) de 3-buten-2-one et 114 mg (1,0 mmol) d'heptanal. Le mélange est agité à reflux, sous courant de diazote, durant 24 h. Le milieu réactionnel devient orangé en fin de réaction. Celui-ci est prélevé pour analyse en GC-MS. La dicétone produite est purifiée par évaporation du solvant puis séparation sur colonne de silice (cyclohexane/AcOEt 8/2). Le rendement est de 92 %.

Les exemples présentés ci-dessous ont été réalisés:

| 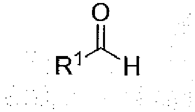 | 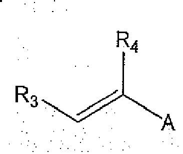 | 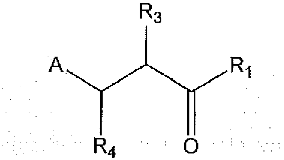 | Rdt % en produit final |
|---|---|---|---|
| 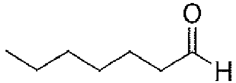 | 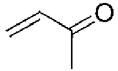 | 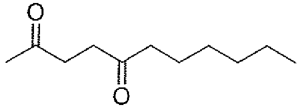 | 92 |
| HCHO | 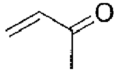 | 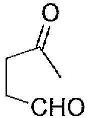 | 35 |
| 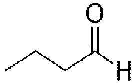 | 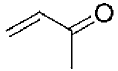 | 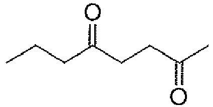 | 74 |
| 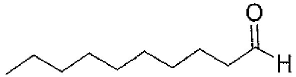 | 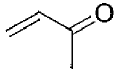 | 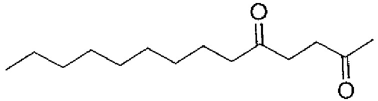 | 66 |
| 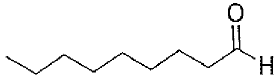 | 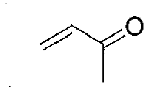 | 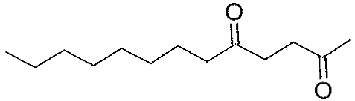 | 69 |
| 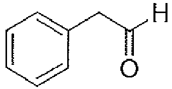 | 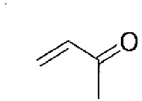 | 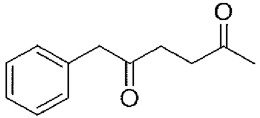 | 74 |

| | | | |
|---|---|---|---|
| 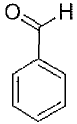 | 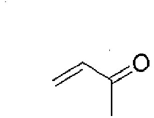 | 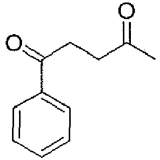 | 27 |
| 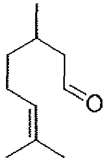 | 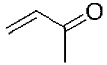 | 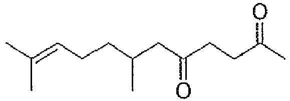 | 39 |
| 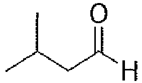 | 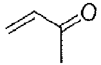 | 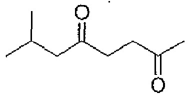 | 73 |
| 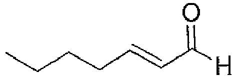 | 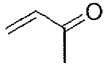 | 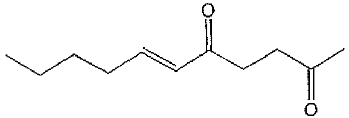 | 49 |
| 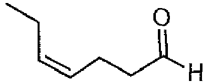 | 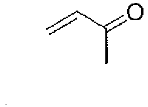 | 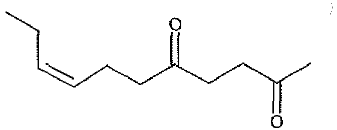 | 64 |
| 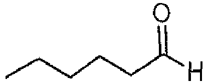 | 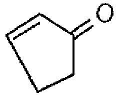 | 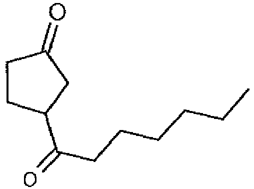 | 31 |

### Exemple 2 : Etude des conditions réactionnelles :

Les conditions de mise en oeuvre de la réaction ont été étudiées en utilisant comme substrat l'heptanal et la 3-butèn-2-one dans les proportions suivantes :

| | m, g | Mr, g/mol | V, mL | n, mol | éq | d, g/mol |
|---|---|---|---|---|---|---|
| heptanal | 0,046 | 114,18 | 0,056 | 0,00040 | 1,00 | 0,82 |
| 3-butèn-2-one | 0,056 | 70,09 | 0,065 | 0,00080 | 2,00 | 0,86 |
| ascorbate de sodium | 0,048 | 198,12 | - | 0,00024 | 0,60 | - |
| thiamine | 0,013 | 337,23 | - | 0,00004 | 0,10 | - |
| 1,2-PrOH/iPrOH | - | - | 0,100 | - | - | - |

### 1. Effet de la température

La réaction a été étudiée à trois températures différentes :

| | Rendement | Conv |
|---|---|---|
| 70°C | 94% | 68% |
| 80°C | 92% | 85% |
| 90°C | 87% | 92% |

Les résultats ont permis de montrer que la conversion augmente avec la température. Le rendement est le meilleur à une température de 80 °C.

### 2. Mode d'addition

La méthode d'addition des vitamines a également été étudiée :

| **Durée** | **T°C** | **Conditions d'introduction** | **% dione** | **% conver sion** |
|---|---|---|---|---|
| **2h** | 80°C | Ajout progressif des vitamines sans solvant (30min/30min) | 92 | 85 |
| **2h** | 90°C | Ajout progressif des vitamines sans solvant (30min/30min) | 87 | 92 |
| **2h** | 80°C | Ajout progressif des vitamines en solution aqueuse (30min/30min) | 71 | 51 |
| **2h** | 80°C | Ajout progressif des vitamines dans iPrOH/H2O (1/1) (30min/30min) | 79 | 85 |
| **2h** | 80°C | Ajout progressif des vitamines dans iPrOH/H2O (4/1) (30min/30min) | 77 | 82 |
| **2h** | 80°C | Ajout progressif de la vitamine B1 dans iPrOH/1,3-PrOH (1/1) (30min/30min) | 87 | 85 |
| **2h** | 80°C | Ajout progressif de la vitamine B1 dans iPrOH/1,3-PrOH (1/1) (15min/15min) | 90 | 84 |
| **2h** | 80°C | Ajout progressif de la vitamine B1 dans iPrOH/1,3-PrOH (1/1) (8 fois avec l'intervalle de 15min) | 94 | 76 |

On observe que l'addition progressive des vitamines sans solvant ou dans un alcool conduit à une conversion et un rendement plus élevés.

### 3. Etude de la quantité de solvant

La réaction entre l'heptanal et la 3-butèn-2-one a été étudiée dans différentes quantités de solvant (2-propanol) :

| | Rdt | Conv | |
|---|---|---|---|
| 0,1 mL pour 0,05 mL d'heptanal | 90% | 84% | 2 x 0,05 éq, 80°C |
| 0,05 mL pour 0,05 mL d'heptanal | 75% | 86% | 2 x 0,05 éq, 80°C |
| 0,05 mL pour 0,05 mL d'heptanal | 84% | 90% | One pot, 80°C |
| 0,025 mL pour 0,05 mL d'heptanal | 83% | 83% | One pot, 80°C |
| 0,05 mL pour 0,05 mL d'heptanal | 72% | 92% | One pot, 70°C |
| 0,05 mL pour 0,05 mL d'heptanal | 92% | 77% | One pot, 90°C |

### 4. Quantité de buténone

L'influence de la proportion de 3-butèn-2-one a été évaluée.

| | Rdt | Conv |
|---|---|---|
| 2 éq | 84% | 90% |
| 4 éq | 72% | 87% |

Les résultats montrent que l'utilisation de quatre équivalents de 3-butèn-2-one par rapport à l'heptanal conduit à un rendement plus faible en dicétone 1,4 que l'utilisation de 2 équivalents.

### Exemple 3 : Condensation acyloine d'heptanal avec des vitamines

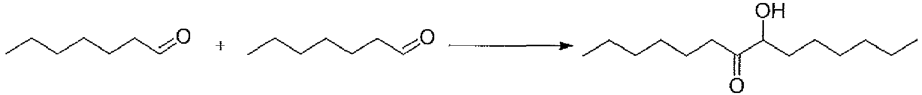

### Mode opératoire :

L'heptanal (0,1 mL, 0,72 mmol), la 3-buten-2-one (0,03 mL, 0,36 mmol), l'ascorbate de sodium (0,086g, 0,43 mmol), la thiamine (0,024g, 0,07 mmol) sont additionnés dans le mélange d'*i*PrOH/1,2-Propandiol (1/1) (0,09 mL) à température ambiante. Puis le mélange réactionnel est agité pendant 3 heures à 80°C sous atmosphère inerte. Au bout de 3 heures le brut réactionnel est analysé en GC/MS, produit désiré est obtenu avec le rendement de 63% avec 57% de conversion (au total 36% de produit dans le brut réactionnel).

### Exemple 4 - Préparation de cyclopenténones à partir des dicétones 1,4 :

### Mode opératoire :

Dans un ballon muni d'un barreau magnétique, d'un réfrigérant et d'une arrivée d'azote, sont introduits 10 mL d'éthanol anhydre, 33,7 mg (0,1 mmol) de chlorhydrate de thiamine et 119 mg (0,6 mmol) d'ascorbate de sodium. L'ensemble est agité à température ambiante durant 10 minutes puis sont ajoutés 140 mg (2,0 mmol) de 3-buten-2-one et 112 mg (1,0 mmol) d'hepten-2-al. Le mélange est agité à reflux, sous courant de diazote, durant 24 h. L'évolution du milieu réactionnel est suivie par analyse en GC-MS. La cyclopenténone obtenue est purifiée par évaporation du solvant puis séparation sur colonne de silice (toluène/AcOEt 9/1). Le rendement est de 51 %.

En utilisant le mode opératoire décrit ci-dessus, les produits suivants ont été obtenus :

| 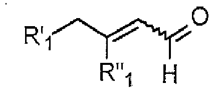 | 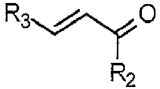 | 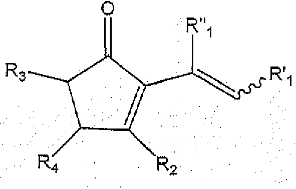 | Rdt % en cyclopenténone |
|---|---|---|---|
| 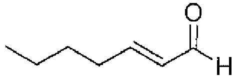 | 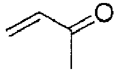 | 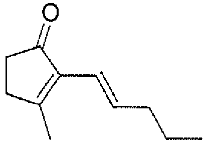 | 51 |
| 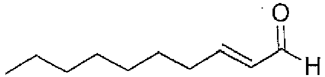 | 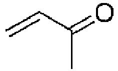 | 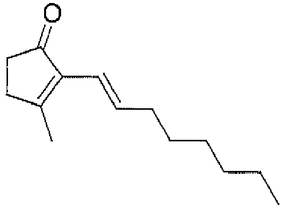 | 49 |
| 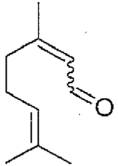 | 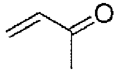 | 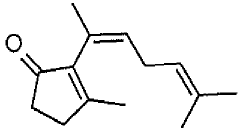 | 19 |
| 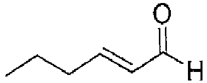 | 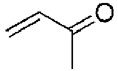 | 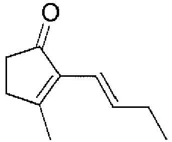 | 89 |

## Revendications

1. Procédé de préparation d'un produit de formule I : 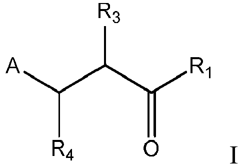 dans laquelle A représente un radical choisi parmi :
-
CO-R₂
-
CO-O-R₂a
-
CN
-
C(O)-N Ra R'a
-
CH- (CO₂Rb)₂
R₁ représente un atome d'hydrogène ou un radical alkyle ou alkylène linéaire ou ramifié ayant au plus 12 atomes de carbone soit R₁ représente un radical cycloalkyle saturé ou insaturé ayant de 3 à 7 atomes de carbone et comportant éventuellement un ou plusieurs hétéroatomes choisis parmi les atomes d'azote, de soufre ou d'oxygène, soit R₁ représente un radical aryle carbocyclique ou hétérocyclique, chacun de ces radicaux alkyle, alkylène, aryle ou cycloalkyle étant éventuellement substitués,
R₂ représente un radical alkyle linéaire ou ramifié ayant de 1 à 12 atomes de carbone éventuellement substitué soit R₂ représente un radical cycloalkyle saturé ou insaturé ayant de 3 à 7 atomes de carbone et comportant éventuellement un ou plusieurs hétéroatomes choisis parmi les atomes d'azote, de soufre ou d'oxygène soit R₂ représente un radical aryle carbocyclique ou hétérocyclique chacun de ces radicaux alkyle, aryle ou cycloalkyle étant éventuellement substitués,
R₂a représente un radical alkyle linéaire ou ramifié ayant de 1 à 12 atomes de carbone
Ra et R'a identiques ou différents sont choisis parmi radicaux alkyle ou alkoxy linéaires ou ramifiés ayant de 1 à 12 atomes de carbone, étant entendu que Ra et R'a ne peuvent pas représenter simultanément chacun un radical alkoxy linéaire ou ramifié ayant de 1 à 12 atomes de carbone
Rb est choisi parmi les radicaux alkyles linéaires ou ramifiés ayant de 1 à 12 atomes de carbone
R₃ représente un atome d'hydrogène, alkyle ou alkylène linéaire ou ramifié ayant au plus 6 atomes de carbone soit R₃ représente un radical aryle carbocyclique ou hétérocyclique, chacun de ces radicaux alkyle, alkylène ou aryle étant éventuellement substitués,
ou les radicaux R₂ et R₃ sont liés entre eux pour former un cycle ayant de 5 à 7 atomes de carbone, ladite chaine comportant éventuellement un ou plusieurs hétéroatomes choisis parmi les atomes d'azote, de soufre ou d'oxygène,
R₄ est un atome d'hydrogène ou est choisi parmi les radicaux alkyle éventuellement substitués, acylamido ayant de 2 à 12 atomes de carbone, carboxylester, de préférence R₄ représente un atome d'hydrogène,
**caractérisé en ce que** l'on fait agir un produit de formule II : 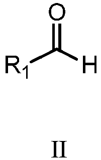 avec un produit de formule III 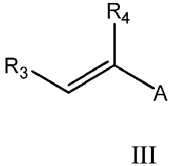
en présence d'une part d'un composé organique comportant au moins un cycle choisi parmi un 1,3-imidazolium, un 1,2,4-triazolium ou un thiazolium et d'autre part d'acide ascorbique sous forme libre ou salifiée d'un composé comprenant un motif 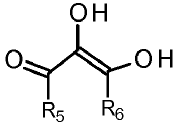
dans lequel R₅ et R₆ représentent indépendamment l'un de l'autre un atome d'hydrogène, un radical alkyle linéaire ou ramifié ayant de 1 à 12 atomes de carbone ou R₅ et R₆ sont liés entre eux pour former un cycle ayant de 3 à 7 chaînons, ledit alkyle ou ledit cycle étant éventuellement substitué par un ou plusieurs hétéroatomes, notamment choisi parmi O, N et S, notamment une réductone, de préférence le 2-hydroxypropanedial ou la 2,3-dihydroxycyclopent-2-en- 1-one ou l'acide croconique dans un solvant organique.

2. Procédé selon la revendication 1, mis en oeuvre avec un produit de formule III dans lequel A est C-OR₂.

3. Procédé selon la revendication 1 ou 2, dans lequel la réaction est mise en oeuvre en présence d'une part d'un composé organique comportant au moins un cycle thiazolium et d'autre part d'acide ascorbique sous forme libre ou salifiée ou d'un composé comprenant un motif 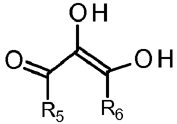 tel que défini à la revendication 1, notamment une réductone, de préférence le 2-hydroxypropanedial ou la 2,3-dihydroxycyclopent-2-en- 1-one ou l'acide croconique dans un solvant organique.

4. Procédé selon la revendication 3, dans lequel la réaction est mise en oeuvre en présence d'une part de la thiamine ou d'un sel de thiamine et d'autre part d'acide ascorbique sous forme libre ou salifiée ou d'un composé comprenant un motif 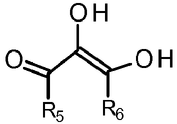 tel que défini à la revendication 1, notamment une réductone, de préférence le 2-hydroxypropanedial ou la 2,3-dihydroxycyclopent-2-en- 1-one ou l'acide croconique, de préférence l'acide ascorbique sous forme libre ou salifiée, dans un solvant organique.

5. Procédé selon l'une des revendications 1 à 4, **caractérisé en ce que** le ou les substituants que peuvent porter les radicaux alkyle ou alkylène linéaire ou ramifié, aryle carbocyclique ou hétérocyclique ou cycloalkyle sont choisis parmi les radicaux aryle carbocyclique ou hétérocyclique, eux-mêmes éventuellement substitués, les radicaux carboxylique libre estérifié ou salifié, le groupement oxo libre sous forme de cétone ou protégé sous forme de cétal, les atomes d'halogène, les radicaux alkoxy ayant de 1 à 6 atomes de carbone tel que méthoxy ou éthoxy.

6. Procédé selon l'une des revendications 1 ou 2 de préparation d'un produit de formule la 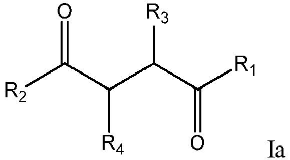
dans laquelle R₁ R₃ et R₄ ont la signification indiquée à la revendication 1 et correspondant à un produit de formule I telle que définie à la revendication 1 dans laquelle A représente un radical CO-R₂ dans lequel R₂ a la signification indiquée à la revendication 1
**caractérisé en ce que** l'on fait agir un produit de formule II tel que défini à la revendication 1 avec un produit de formule IIIa : 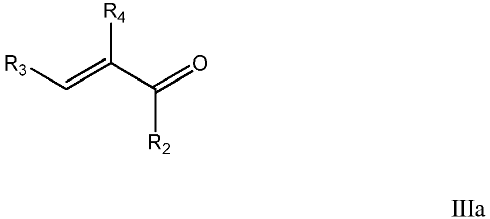 de préférence en présence de thiamine ou d'un sel de thiamine de préférence le chlorhydrate de thiamine et d'acide ascorbique sous forme libre ou salifiée de préférence l'ascorbate de sodium dans un solvant organique.

7. Procédé selon la revendication 3 **caractérisé en ce que** le substituant R₁ est choisi parmi un atome d'hydrogène ou un radical alkyle linéaire ou ramifié ayant de 1 à 9 atomes de carbone, de préférence le n-propyle, le butyle, l'isobutyle, ou le tert-butyle l'hexyle, l'octyle, le nonyle ou un radical alkylène linéaire ou ramifié ayant au plus 9 atomes de carbone de préférence le 1-pentényle, le 1-hexényle, le 2,6-di méthyl 5-heptényle, le 2,6-di méthyl -1,5 diényl heptyle, le 1-nonényle, un radical benzyle ou un radical phényle R₂ représente un radical méthyle et R₃ représente un atome d'hydrogène.

8. Procédé de préparation d'un composé de formule III' : 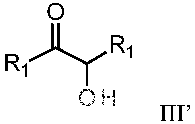 **caractérisé en ce que** l'on fait agir un produit de formule II : 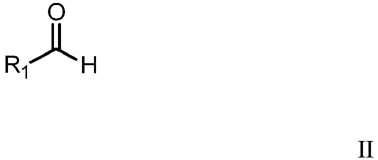 dans laquelle :
R₁ représente un radical alkyle ou alkylène linéaire ou ramifié, ayant au plus 12 atomes de carbone soit R₁ représente un radical cycloalkyle saturé ou insaturé ayant de 3 à 7 atomes de carbone et comportant éventuellement un ou plusieurs hétéroatomes choisis parmi les atomes d'azote, de soufre ou d'oxygène, soit R₁ représente un radical aryle carbocyclique ou hétérocyclique, chacun de ces radicaux alkyle, alkylène, aryle ou cycloalkyle étant éventuellement substitués,
en présence d'une part d'un sel de thiazolium, d'un sel d'1,3-imidazolium ou d'un sel de 1,2,4-triazolium, tel que défini à l'une des revendications 1 à 3, et d'autre part d'acide ascorbique sous forme libre ou salifiée, ou d'un composé comprenant un motif 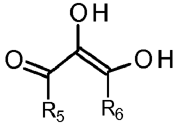 tel que défini à la revendication 1, notamment une réductone, de préférence le 2-hydroxypropanedial ou la 2,3-dihydroxycyclopent-2-en- 1-one ou l'acide croconique dans un solvant organique.

9. Procédé selon la revendication 8, en présence de vitamine B1 et d'acide ascorbique sous forme libre ou salifiée.

10. Procédé de préparation d'un produit de formule IVi 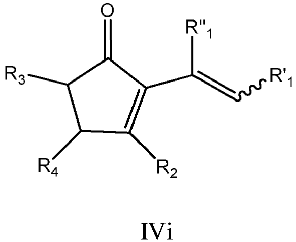
R₂, R₃ et R₄ ont la signification indiquée à la revendication 1,
dans laquelle R'₁ représente un radical alkyle ou alkylène linéaire ou ramifié ayant au plus 12 atomes de carbone, éventuellement substitué par un radical aryle carbocyclique ou hétérocyclique, un radical -CHO, un radical carboxylique libre estérifié ou salifié, un groupement oxo, soit R'₁ représente un radical aryle carbocyclique ou hétérocyclique, R"₁ représente un atome d'hydrogène ou radical alkyle ayant au plus 6 atomes de carbone **caractérisé en ce que** l'on fait agir un produit de formule IIi 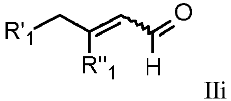 avec un produit de formule IIIa : 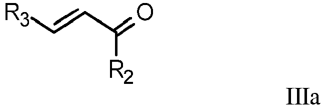 tel que défini à la revendication 1 ou 2 en présence de thiamine ou d'un sel de thiamine, de préférence le chlorhydrate de thiamine et d'acide ascorbique sous forme libre ou salifiée de préférence l'ascorbate de sodium dans un solvant organique pour obtenir un produit de formule Ii : 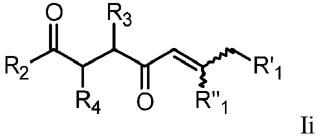 produit de formule Ii qui se transforme spontanément en produit de formule IVi en présence de thiamine ou d'un sel de thiamine, de préférence le chlorhydrate de thiamine et d'acide ascorbique sous forme libre ou salifiée de préférence l'ascorbate de sodium.

11. Utilisation d'un mélange comprenant d'une part un composé organique comportant au moins un cycle choisi parmi un 1,3-imidazolium, un 1,2,4-triazolium ou un thiazolium et d'autre part l'acide ascorbique sous forme libre ou salifiée pour la préparation de composés 1,4-dicarbonylés linéaires, de préférence les produits de formules I, Ia, I'a, I"a ou Ii ou de cyclopenténones, tels que la dihydrojasmone et ses dérivés de préférence les produits de formules IV', IV" ou IVi, tels que définis dans les revendications 1 à 9, de préférence dans une réaction de type Stetter.

12. Utilisation selon la revendication 11, d'un mélange comprenant d'une part un composé organique comportant au moins un cycle thiazolium, de préférence la vitamine B1 ou un sel de thiamine et d'autre part l'acide ascorbique sous forme libre ou salifiée.

13. Utilisation selon l'une des revendications 11 à 12, dans laquelle l'acide ascorbique sous forme libre ou salifiée est en excès par rapport à la thiamine ou au sel de thiamine, notamment dans un ratio de 2/1 à 20/1, avantageusement de 2/1 à 10/1, plus avantageusement de 3/1 à 7/1, de préférence de 5/1 ou 6/1.

## Patentansprüche

1. Verfahren zur Herstellung eines Produkts von Formel I, umfassend: 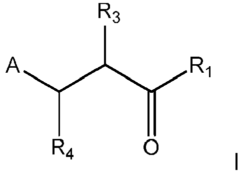 wobei A ein Radikal darstellt, das ausgewählt ist aus:
-
CO-R₂
-
CO-O-R₂a
-
CN
-
C(O)-N Ra R'a
-
CH- (CO₂Rb)₂
R₁ ein Wasserstoffatom oder ein lineares oder verzweigtes Alkyl- oder Alkylenradikal darstellt, das höchstens 12 Kohlenstoffatome aufweist, oder R₁ ein gesättigtes oder ungesättigtes Cycloalkylradikal darstellt, das 3 bis 7 Kohlenstoffatome aufweist und optional umfassend ein oder mehrere Heteroatome, ausgewählt aus Stickstoff-, Schwefel- oder Sauerstoffatomen, oder R₁ ein carbocyclisches oder heterocyclisches Arylradikal darstellt, wobei jedes dieser Alkyl-, Alkylen-, Aryl- oder Cycloalkylradikale optional substituiert ist,
R₂ ein lineares oder verzweigtes Alkylradikal darstellt, das 1 bis 12 Kohlenstoffatome aufweist, die optional substituiert sind, oder R₂ ein gesättigtes oder ungesättigtes Cycloalkylradikal darstellt, das 3 bis 7 Kohlenstoffatome aufweist und optional umfassend ein oder mehrere Heteroatome, ausgewählt aus Stickstoff-, Schwefel- oder Sauerstoffatomen, oder R₂ ein carbocyclisches oder heterocyclisches Arylradikal darstellt, wobei jedes dieser Alkyl-, Aryl- oder Cycloalkylradikale optional substituiert ist,
R₂a ein lineares oder verzweigtes Alkylradikal darstellt, das 1 bis 12 Kohlenstoffatome aufweist
Ra und R'a, die identisch oder verschieden sind, ausgewählt sind aus lineares oder verzweigtes Alkyl- oder Alkoxyradikalen, die 1 bis 12 Kohlenstoffatome aufweisen, wobei Ra und R'a nicht gleichzeitig jeweils ein lineares oder verzweigtes Alkoxyradikal darstellen können, das 1 bis 12 Kohlenstoffatome aufweist
Rb ausgewählt ist aus linearen oder verzweigten Alkylradikalen, die 1 bis 12 Kohlenstoffatome aufweisen
R₃ ein Wasserstoffatom, ein lineares oder verzweigtes Alkyl oder Alkylen darstellt, das höchstens 6 Kohlenstoffatome aufweist, oder R₃ ein carbocyclisches oder heterocyclisches Arylradikal darstellt, wobei jeder dieser Alkyl-, Alkylen- oder Arylradikale optional substituiert ist,
oder die Radikale R₂ und R₃ miteinander verbunden sind, um einen Ring zu bilden, der 5 bis 7 Kohlenstoffatome aufweist, wobei die Kette optional ein oder mehrere Heteroatome umfasst, die ausgewählt sind aus Stickstoff-, Schwefel- oder Sauerstoffatomen,
R₄ ein Wasserstoffatom ist oder ausgewählt ist aus optional substituiertem Alkyl, Acylamido, das 2 bis 12 Kohlenstoffatome aufweist, Carboxylester, wobei R₄ vorzugsweise ein Wasserstoffatom darstellt,
**dadurch gekennzeichnet, dass** ein Produkt von Formel II einwirken gelassen wird: 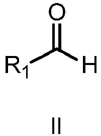 mit einem Produkt von Formel III 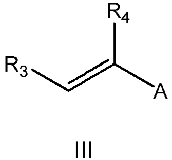 in Anwesenheit einerseits einer organischen Verbindung, umfassend mindestens einen Ring, ausgewählt aus einem 1,3-Imidazolium, einem 1,2,4-Triazolium oder einem Thiazolium, und andererseits von Ascorbinsäure in freier oder in Salzform einer Verbindung umfassend ein Motiv 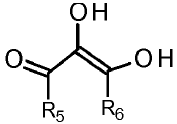 wobei R₅ und R₆ unabhängig voneinander ein Wasserstoffatom, ein lineares oder verzweigtes Alkylradikal darstellt, das 1 bis 12 Kohlenstoffatome aufweist oder R₅ und R₆ miteinander verbunden sind, um einen 3- bis 7-gliedrigen Ring zu bilden, wobei das Alkyl oder der Ring optional durch ein oder mehrere Halogenatome substituiert ist, insbesondere ausgewählt aus O, N und S, insbesondere ein Redukton, vorzugsweise 2-Hydroxypropandial oder 2,3-Dihydroxycyclopent-2-en-1-on oder Krokonsäure in einem organischen Lösungsmittel.

2. Verfahren nach Anspruch 1, durchgeführt mit einem Produkt von Formel III, wobei A C-OR₂ ist.

3. Verfahren nach Anspruch 1 oder 2, wobei die Reaktion durchgeführt in Anwesenheit einerseits einer organischen Verbindung umfassend mindestens einen Thiazoliumring und andererseits von Ascorbinsäure in freier oder in Salzform oder einer Verbindung umfassend ein Motiv 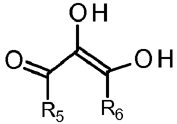 wie definiert in Anspruch 1, insbesondere ein Redukton, vorzugsweise 2-Hydroxypropandial oder 2,3-Dihydroxycyclopent-2-en- 1-on oder Krokonsäure in einem organischen Lösungsmittel.

4. Verfahren nach Anspruch 3, wobei die Reaktion in Anwesenheit von Thiamin oder einem Thiaminsalz einerseits und Ascorbinsäure andererseits 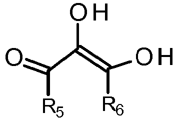 in freier oder in Salzform oder einer Verbindung durchgeführt wird, umfassend ein Motiv wie definiert in Anspruch 1, insbesondere ein Redukton, vorzugsweise 2-Hydroxypropandial oder 2,3-Dihydroxycyclopent-2-en-1-on oder Krokonsäure, vorzugsweise Ascorbinsäure in freier Form oder in Salzform, in einem organischen Lösungsmittel.

5. Verfahren nach einem der Ansprüche 1 bis 4, **dadurch gekennzeichnet, dass** der oder die Substituenten, die die linearen oder verzweigten Alkyl- oder Alkylenradikale, die carbocyclischen oder heterocyclischen Aryl- oder Cycloalkylradikale tragen können, ausgewählt sind aus carbocyclischen oder heterocyclischen Aryl- oder Cycloalkylradikalen, die ihrerseits optional substituiert sind, veresterte oder in ein Salz überführte freie Carboxylradikale, einer freien Oxogruppierung in Form eines Ketons oder geschützt in Form eines Ketals, Halogenatomen, Alkoxyradikalen, die 1 bis 6 Kohlenstoffatome, wie z. B. Methoxy oder Ethoxy, aufweisen.

6. Verfahren nach einem der Ansprüche 1 oder 2 zur Herstellung eines Produkts von Formel la 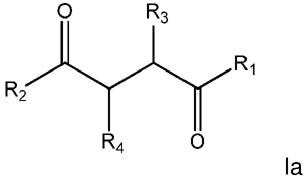
wobei R₁, R₃ und R₄ die in Anspruch 1 angegebene Bedeutung haben, und einem Produkt von Formel I wie definiert in Anspruch 1 entsprechen, wobei A ein Radikal CO-R₂ darstellt, wobei R₂ die in Anspruch 1 angegebene Bedeutung hat.
**dadurch gekennzeichnet, dass** ein Produkt von Formel II wie definiert in Anspruch 1 mit einem Produkt der Formel IIIa einwirken gelassen wird: 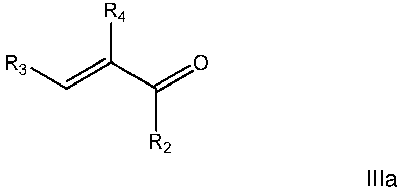 vorzugsweise in Anwesenheit von Thiamin oder einem Thiaminsalz vorzugsweise Thiaminhydrochlorid und Ascorbinsäure in freier oder in Salzform vorzugsweise Natriumascorbat in einem organischen Lösungsmittel.

7. Verfahren nach Anspruch 3, **dadurch gekennzeichnet, dass** der Substituent R₁ ausgewählt ist aus einem Wasserstoffatom oder einem linearen oder verzweigten Alkylradikal, das 1 bis 9 Kohlenstoffatome aufweist, vorzugsweise n-Propyl, Butyl, Isobutyl oder tert-Butyl, Hexyl, Octyl, Nonyl oder einem linearen oder verzweigten Alkylenradikal, das höchstens 9 Kohlenstoffatome aufweist, vorzugsweise 1-Pentenyl, 1-Hexenyl, 2,6-Dimethyl-5-heptenyl, 2,6-Dimethyl-1,5-dienyl-heptyl, 1-Nonenyl, einem Benzylradikal oder einem Phenylradikal
R₂ ein Methylradikal darstellt und R₃ ein Wasserstoffatom darstellt.

8. Verfahren zur Herstellung einer Verbindung von Formel III': 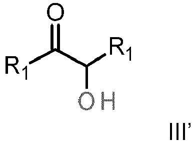 **dadurch gekennzeichnet, dass** ein Produkt von Formel II einwirken gelassen wird: 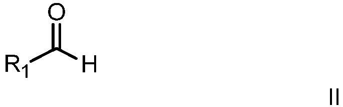 wobei:
R₁ ein lineares oder verzweigtes Alkyl- oder Alkylenradikal darstellt, das höchstens 12 Kohlenstoffatome aufweist, oder R₁ ein gesättigtes oder ungesättigtes Cycloalkylradikal darstellt, das 3 bis 7 Kohlenstoffatome aufweist und optional umfassend ein oder mehrere Heteroatome, ausgewählt aus Stickstoff-, Schwefel- oder Sauerstoffatomen, oder R₁ ein carbocyclisches oder heterocyclisches Arylradikal darstellt, wobei jedes dieser Alkyl-, Alkylen-, Aryl- oder Cycloalkylradikale optional substituiert ist,
in Anwesenheit einerseits eines Thiazoliumsalzes, eines 1,3-lmidazoliumsalzes oder eines 1,2,4-Triazoliumsalzes wie definiert in einem der Ansprüche 1 bis 3, und andererseits von Ascorbinsäure in freier Form oder in Salzform oder einer Verbindung, umfassend ein Motiv 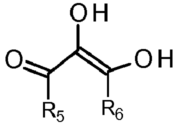 wie definiert in Anspruch 1, insbesondere ein Redukton, vorzugsweise 2-Hydroxypropandial oder 2,3-Dihydroxycyclopent-2-en- 1-on oder Krokonsäure in einem organischen Lösungsmittel.

9. Verfahren nach Anspruch 8, in Anwesenheit von Vitamin B1 und Ascorbinsäure in freier oder in Salzform.

10. Verfahren zur Herstellung eines Produkts von Formel IVi 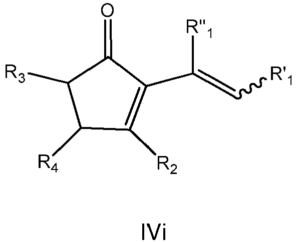
wobei R₂, R₃ und R₄ die in Anspruch 1 angegebene Bedeutung haben,
wobei R'₁ einen lineares oder verzweigtes Alkyl- oder Alkylenradikal darstellt, das höchstens 12 Kohlenstoffatome aufweist, optional substituiert durch ein carbocyclisches oder heterocyclisches Arylradikal, ein -CHO-Radikal, ein verestertes oder in ein Salz überführtes freies Carboxylradikal, eine Oxogruppierung, wobei entweder R'₁ ein carbocyclisches oder heterocyclische Arylradikal darstellt, R"₁ ein Wasserstoffatom oder ein Alkylradikal darstellt, das höchstens 6 Kohlenstoffatomen aufweist
**dadurch gekennzeichnet, dass** ein Produkt von Formel IIi einwirken gelassen wird: mit einem Produkt von Formel IIIa: 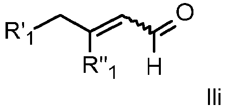 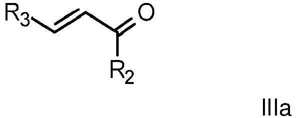 wie definiert in Anspruch 1 oder 2 in Anwesenheit von Thiamin oder einem Thiaminsalz, vorzugsweise Thiaminhydrochlorid, und Ascorbinsäure in freier oder in Salzform, vorzugsweise Natriumascorbat, in einem organischen Lösungsmittel, um ein Produkt von Formel li zu erlangen: 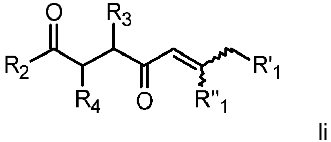 Produkt von Formel li, das sich in Anwesenheit von Thiamin oder einem Thiaminsalz, vorzugsweise Thiaminhydrochlorid, und Ascorbinsäure in freier oder in Salzform, vorzugsweise Natriumascorbat, spontan in das Produkt von Formel IVi umwandelt.

11. Verwendung eines Gemischs, umfassend einerseits eine organische Verbindung mit mindestens einem Ring, ausgewählt aus einem 1,3-Imidazolium, einem 1,2,4-Triazolium oder einem Thiazolium, und andererseits Ascorbinsäure in freier Form oder als Salz enthält, zur Herstellung von linearen 1,4-Dicarbonylverbindungen, vorzugsweise von Produkten von Formel I, la, l'a, I"a oder li oder Cyclopentenone, wie Dihydrojasmon und seine Derivate vorzugsweise die Produkte von Formel IV', IV" oder IVi, wie definiert in den Ansprüchen 1 bis 9, vorzugsweise in einer Reaktion vom Stetter-Typ.

12. Verwendung eines Gemischs nach Anspruch 11, umfassend einerseits eine organische Verbindung mit mindestens einem Thiazoliumring, vorzugsweise Vitamin B1 oder ein Thiaminsalz, und andererseits Ascorbinsäure in freier oder in Salzform.

13. Verwendung nach einem der Ansprüche 11 bis 12, wobei die Ascorbinsäure in freier oder in Salzform im Verhältnis zu dem Thiamin oder Thiaminsalz im Überschuss ist, insbesondere in einem Verhältnis von 2:1 bis 20:1, vorteilhafterweise von 2:1 bis 10:1, noch vorteilhafter von 3:1 bis 7:1, vorzugsweise von 5:1 oder 6:1.

## Claims

1. Method for the preparation of a formula I product: 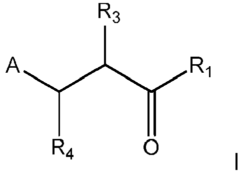 in which A represents a radical selected from:
-
CO-R₂
-
CO-O-R₂a
-
CN
-
C(O)-N Ra R'a
-
CH- (CO₂Rb)₂
R₁ represents a hydrogen atom or a linear or branched alkyl or alkylene radical having at most 12 carbon atoms or R₁ represents a saturated or unsaturated cycloalkyl radical having from 3 to 7 carbon atoms and possibly containing one or more heteroatoms chosen from nitrogen, sulphur or oxygen atoms, or R₁ represents a carbocyclic or heterocyclic aryl radical, each of these alkyl, alkylene or heterocyclic radicals being possibly substituted,
R₂ represents a linear or branched alkyl radical having from 1 to 12 carbon atoms, possibly substituted, or R₂ represents a saturated or unsaturated cycloalkyl radical having from 3 to 7 carbon atoms and possibly containing one or more heteroatoms chosen from nitrogen, sulphur or oxygen atoms, or R₂ represents a carbocyclic or heterocyclic aryl radical, each of these alkyl, aryl or heterocyclic radicals being possibly substituted,
R₂a represents a linear or branched alkyl radical having from 1 to 12 carbon atoms.
Ra and R'a, which may be identical or different, are chosen from linear or branched alkyl or alkoxy radicals having from 1 to 12 carbon atoms, it being understood that Ra and R'a cannot simultaneously represent a linear or branched alkoxy radical having from 1 to 12 carbon atoms.
Rb is selected from linear or branched alkyl radicals having from 1 to 12 carbon atoms.
R₃ represents a hydrogen atom, linear or branched alkyl or alkylene having at most 6 carbon atoms or R₃ represents a carbocyclic or heterocyclic aryl radical, each of these alkyl, alkylene or aryl radicals being possibly substituted,
or the radicals R₂ and R₃ are linked together to form a ring having from 5 to 7 carbon atoms, said chain possibly including one or more heteroatoms selected from nitrogen, sulphur or oxygen atoms,
R₄ is hydrogen or is selected from possibly substituted alkyl, acylamido with 2 to 12 carbon atoms, carboxylester, preferably R₄ is hydrogen,
**characterised in that** a product of the formula II: with a formula III product 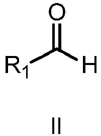 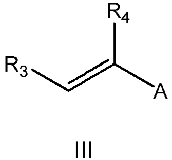 in the presence on the one hand of an organic compound comprising at least one ring chosen from a 1,3-imidazolium, a 1,2,4-triazolium or a thiazolium and on the other hand ascorbic acid in free or salified form of a compound comprising a 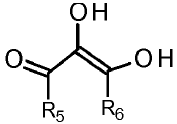
in which R₅ and R₆, independently of each other, represent a hydrogen atom, a linear or branched alkyl radical having from 1 to 12 carbon atoms or R₅ and R₆ are linked together to form a 3- to 7-membered ring, the said alkyl or the said ring being possibly substituted by one or more heteroatoms, in particular chosen from O, N and S, in particular a reductone, preferably 2-hydroxypropanedial or 2,3- dihydroxycyclopentadial -2-en-1-one or croconic acid in an organic solvent.

2. Method according to claim 1, carried out with a formula III product wherein A is COR₂.

3. Process according to claim 1 or 2, in which the reaction is carried out in the presence of, on the one hand, an organic compound containing at least one thiazolium ring and, on the other hand, ascorbic acid in free or salified form or a compound comprising a 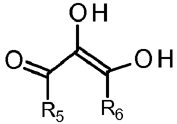 motive as defined in claim 1, in particular a reductone, preferably 2-hydroxypropanedial or 2,3-dihydroxycyclopent-2-en-1-one or croconic acid in an organic solvent.

4. Method according to claim 3, wherein the reaction is carried out on one hand in the presence of thiamine or a thiamine salt and ascorbic acid on the other hand 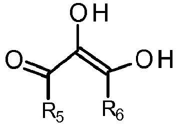 in free or salified form or a compound comprising a unit as defined in claim 1, in particular a reductone, preferably 2-hydroxypropanedial or 2,3-dihydroxycyclopent-2-en-1-one or croconic acid, preferably ascorbic acid in free or salified form, in an organic solvent.

5. Method according to any of claims 1 to 4, **characterised in that** the substituent(s) which may be carried by the linear or branched alkyl or alkylene radicals, carbocyclic or heterocyclic aryl radicals or cycloalkyl radicals are chosen from carbocyclic or heterocyclic aryl radicals, themselves possibly substituted, the free esterified or salified carboxylic radicals, the free oxo group in the form of a ketone or protected in the form of a ketal, the halogen atoms, the alkoxy radicals having from 1 to 6 carbon atoms such as methoxy or ethoxy.

6. Method according to any of claims 1 or 2 for the preparation of a product of formula la 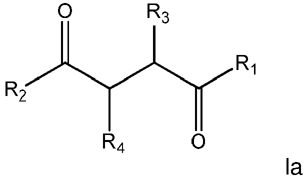
in which R₁, R₃ and R₄ have the meaning given in claim 1 and corresponding to a product of formula I as defined in claim 1 in which A represents a CO-R₂ radical in which R₂ has the meaning given in claim 1
**characterised in that** a product of formula II as defined in claim 1 is reacted with a product of formula IIIa: 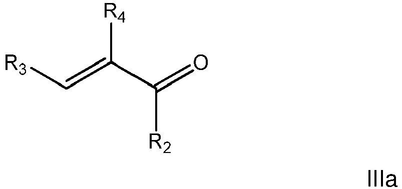 preferably in the presence of thiamine or a thiamine salt, preferably thiamine hydrochloride, and ascorbic acid in free or salified form, preferably sodium ascorbate in an organic solvent.

7. Process according to claim 3, **characterized in that** the substituent R₁ is chosen from a hydrogen atom or a linear or branched alkyl radical having from 1 to 9 carbon atoms, preferably n-propyl, butyl, isobutyl, or tert-butyl, hexyl, octyl, nonyl or a linear or branched alkyl radical having at most 9 carbon atoms, preferably 1-pentenylor, 1-hexenyl, 2,6-di methyl-5-heptenyl, 2,6-di methyl-1,5-dienyl heptyl, 1-nonenyl, a benzyl radical or a phenyl radical
R₂ represents a methyl radical and R₃ represents a hydrogen atom.

8. Method for the preparation of a formula III' compound: 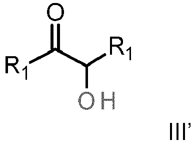 **characterised in that** it is a formula II product: 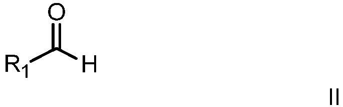 in which:
R₁ represents a linear or branched alkyl or alkylene radical having at most 12 carbon atoms or R₁ represents a saturated or unsaturated cycloalkyl radical having from 3 to 7 carbon atoms and optionally comprising one or more heteroatoms chosen from nitrogen, sulphur or oxygen atoms, or R₁ represents a carbocyclic or heterocyclic aryl radical, each of these alkyl, alkylene, aryl or cycloalkyl radicals being possibly substituted,
in the presence of a thiazolium salt, an 1,3-imidazolium salt or a 1,2,4-triazolium salt, as defined in one of claims 1 to 3, on the one hand, and ascorbic acid in free or salified form on the other hand, or a compound comprising a motive 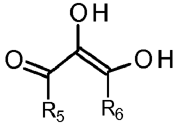 as defined in claim 1, in particular a reductone, preferably 2-hydroxypropanedial or 2,3-dihydroxycyclopent-2-en-1-one or croconic acid in an organic solvent.

9. The process according to claim 8, in the presence of vitamin B1 and ascorbic acid in free or salified form.

10. Method for the preparation of a formula IVi product: 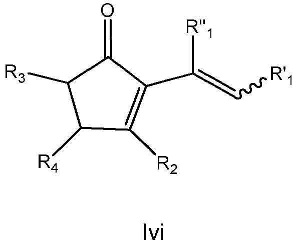
R₂ R₃ and R₄ have the meaning given in claim 1,
in which R'₁ represents a linear or branched alkyl or alkylene radical having at most 12 carbon atoms, possibly substituted by a carbocyclic or heterocyclic aryl radical, a -CHO radical, an esterified or salified free carboxylic radical, an oxo group, or R'₁ represents a carbocyclic or heterocyclic aryl radical, R"₁ represents a hydrogen atom or an alkyl radical having at most 6 carbon atoms
**characterised in that** it is a formula IIi product with a formula IIIa product: 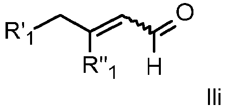 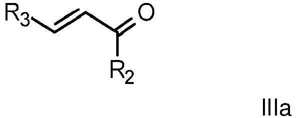 as defined in claim 1 or 2 in the presence of thiamine or a thiamine salt, preferably thiamine hydrochloride, and ascorbic acid in free or salified form, preferably sodium ascorbate, in an organic solvent to obtain a formula li product: 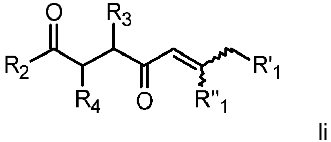 a formula li product which spontaneously converts to a formula Ivi product in the presence of thiamine or a thiamine salt, preferably thiamine hydrochloride and ascorbic acid in free or salified form, preferably sodium ascorbate.

11. Use of a mixture comprising, on the one hand, an organic compound containing at least one ring chosen from a 1,3-imidazolium, a 1,2,4-triazolium or a thiazolium and, on the other hand, ascorbic acid in free or salified form for the preparation of linear 1,4-dicarbonyl compounds, preferably the formulae I, la, I'a, I"a or li products or cyclopentenones, such as dihydrojasmone and its derivatives, preferably the formulae IV', IV" or IVi products, as defined in claims 1 to 9, preferably in a Stetter-type reaction.

12. Use according to claim 11 of a mixture comprising on the one hand an organic compound containing at least one thiazolium ring, preferably vitamin B1 or a thiamine salt, and on the other hand ascorbic acid in free or salified form.

13. Use according to one of claims 11 to 12, wherein the ascorbic acid in free or salified form is in excess with respect to the thiamine or thiamine salt, in particular in a ratio of 2/1 to 20/1, advantageously 2/1 to 10/1, more advantageously 3/1 to 7/1, preferably 5/1 or 6/1.
